# EUROPEAN PATENT APPLICATION

(11) **EP 4 640 234 A1**
(43) Date of publication of application: **29.10.2025**
(21) Application number: 23907880.1
(22) Date of filing: 22.12.2023
(51) Int. Cl.: A61K 38/26, A61K 47/68, A61K 31/56, A61K 45/06, A61P 37/06, C07K 14/605

(54) **PHARMACEUTICAL COMPOSITION FOR PREVENTING OR TREATING GRAFT-VERSUS-HOST DISEASE CONTAINING GLP-2 DERIVATIVE OR LONG-ACTING CONJUGATE THEREOF**

(30) Priority: 23.12.2022 KR 20220183262; 25.08.2023 KR 20230112137; 25.08.2023 KR 20230112138
(71) Applicant: Hanmi Pharm. Co., Ltd., Hwaseong-si, Gyeonggi-do 18536 (KR)
(72) Inventor: KIM, Won Ki, Hwaseong-si, Gyeonggi-do 18469 (KR); PARK, Cho Rong, Hwaseong-si, Gyeonggi-do 18469 (KR); YE, Byeong Jin, Hwaseong-si, Gyeonggi-do 18469 (KR); CHOI, Jae Hyuk, Hwaseong-si, Gyeonggi-do 18469 (KR)
(74) Representative: Mewburn Ellis LLP
(86) International application number: PCT/KR2023/021455
(87) International publication number: WO 2024/136597

(57) **Abstract**

The present invention relates to a prophylactic or therapeutic use of a GLP-2 derivative and/or a long-acting conjugate thereof for graft-versus-host disease. The present invention also relates to a prophylactic or therapeutic use of a GLP-2 derivative and/or a long-acting conjugate thereof for steroid-refractory graft-versus-host disease to use the GLP-2 derivative and/or a long-acting conjugate thereof in combination with steroid.

## Description

### [Technical Field]

The present invention relates to a prophylactic or therapeutic use of a GLP-2 derivative and/or a long-acting conjugate thereof for graft-versus-host disease. The present invention also relates to a prophylactic or therapeutic use of a GLP-2 derivative and/or a long-acting conjugate thereof for steroid-refractory graft-versus-host disease to use the GLP-2 derivative and/or a long-acting conjugate thereof in combination with steroid.

### [Background Art]

Graft-versus-host disease (GVHD) is a disease in which an immune response occurs when T lymphocytes in the peripheral blood or bone marrow of the donor, injected during allogeneic transplantation, recognize the body of the host (i.e., the patient who has received the allogeneic transplantation) as foreign and attack the body. In other words, graft-versus-host disease (GVHD) is caused by injected living lymphocytes and results in symptoms such as liver dysfunction, skin lesions, jaundice, diarrhea, fever, and pancytopenia due to an immune response. In severe cases, the patient may die.

Several methods have been proposed to treat graft-versus-host disease. Methods include removing T cells from transplanted bone marrow cells, administering antibodies to CD80, CD86, and similar targets to suppress the reaction between T cells and antigen-presenting cells, and administering antibodies to cytokines such as IL-2 and IFN-gamma. Additionally, administering immunosuppressant compounds, such as cyclosporine A, rapamycin, FK-506, and steroid preparations, is also used. Among these methods, the administration of immunosuppressant compounds that suppress T cell activation is the most widely used.

In particular, steroid-refractory graft-versus-host disease is fatal in transplant recipients because of direct organ damage from the graft-versus-host disease itself and opportunistic infections caused by the potent immunosuppressants used to treat the disease. As a conventional treatment for acute graft-versus-host disease that does not respond to standard doses of steroids, high-dose steroids, monoclonal or polyclonal antibodies, immunotoxins, and the like have been tried in various ways, but the response rate and survival rate are still low. According to previous reports, the percentage of cases showing improvement beyond a partial response is approximately 60% but the survival rate is 35% when high-dose steroids are used as second-line treatment in patients with steroid-refractory graft-versus-host disease. Therefore, this indirectly suggests that survival is difficult in cases where there is no response to high-dose steroids used as second-line treatment for steroid-refractory graft-versus-host disease.

Recently, ruxolitinib, a JAK1/2 inhibitor, has been approved by the FDA as a therapeutic agent for steroid-refractory graft-versus-host disease (SR-GVHD) that has not responded adequately to steroids. Although a large number of immunosuppressant compounds have been developed to date, cyclosporine A has exhibited the most favorable clinical efficacy and is widely used for organ transplant rejection, including acute graft-versus-host disease.

However, although cyclosporine A can completely suppress T cell activation and treat the disease when used in high doses, cyclosporine A has the problem of having significant side effects, including renal toxicity.

### [Disclosure]

### [Technical Problem]

There is a demand for the development of new drugs that can prevent or treat graft-versus-host disease or steroid-refractory graft-versus-host disease.

### [Technical Solution]

An object of the present invention is to provide a composition for the prevention, improvement, or treatment of graft-versus-host disease, containing a glucagon-like peptide-2 (GLP-2) derivative and/or a long-acting conjugate thereof.

Another object of the present invention is to provide a method for preventing, improving, or treating graft-versus-host disease, the method including administering a GLP-2 derivative, a long-acting conjugate thereof, and/or a composition containing the same to a subject in need thereof.

Still another object of the present invention is to provide a use of a GLP-2 derivative, a long-acting conjugate thereof, and/or a composition containing the same in the manufacture of a medicament for the prevention or treatment of graft-versus-host disease.

Still another object of the present invention is to provide a composition for the prevention, improvement, or treatment of steroid-refractory graft-versus-host disease, containing a GLP-2 derivative, a long-acting conjugate thereof, and/or a composition containing the same.

Still another object of the present invention is to provide a method for preventing, improving, or treating steroid-refractory graft-versus-host disease, the method including administering a GLP-2 derivative, a long-acting conjugate thereof, and/or a composition containing the same to a subject in need thereof.

Still another object of the present invention is to provide a use of a GLP-2 derivative, a long-acting conjugate thereof, and/or a composition containing the same in the manufacture of a medicament for the prevention or treatment of steroid-refractory graft-versus-host disease.

Still another object of the present invention is to provide a therapy for the prevention, improvement, or treatment of steroid-refractory graft-versus-host disease, including using a GLP-2 derivative, a long-acting conjugate thereof, and/or a composition containing the same in combination with a steroid.

Still another object of the present invention is to provide a pharmaceutical composition for the prevention or treatment of steroid-refractory graft-versus-host disease, containing a GLP-2 derivative and/or a long-acting conjugate thereof, in which the pharmaceutical composition administers a GLP-2 derivative and/or a long-acting conjugate thereof in combination with a steroid.

Still another object of the present invention is to provide a combination containing a GLP-2 derivative and/or a long-acting conjugate thereof and a steroid.

Still another object of the present invention is to provide a pharmaceutical composition for the prevention, improvement, or treatment of steroid-refractory graft-versus-host disease, containing the combination.

Still another object of the present invention is to provide a pharmaceutical kit for the prevention, improvement, or treatment of steroid-refractory graft-versus-host disease, containing a GLP-2 derivative and/or a long-acting conjugate thereof and a steroid.

Still another object of the present invention is to provide a method for preventing, improving, or treating steroid-refractory graft-versus-host disease, the method including administering and/or using the combination, pharmaceutical composition or pharmaceutical kit to a subject in need thereof.

Still another object of the present invention is to provide a method for preventing, improving, or treating steroid-refractory graft-versus-host disease, the method including administering in combination and/or using in combination a pharmaceutically effective amount of a GLP-2 derivative, a long-acting conjugate thereof, and/or a composition containing the same and a pharmaceutically effective amount of a composition containing a steroid or both of these to a subject in need thereof.

Still another object of the present invention is to provide a use of the combination, pharmaceutical composition, or pharmaceutical kit for the prevention, improvement, or treatment of steroid-refractory graft-versus-host disease; and/or a use of the combination, pharmaceutical composition, or pharmaceutical kit in the manufacture of a medicament for the prevention, improvement, or treatment of steroid-refractory graft-versus-host disease.

### [Advantageous Effects]

A GLP-2 derivative, a long-acting conjugate thereof, or a composition containing the same of the present invention can be applied to the prevention, treatment, and improvement of graft-versus-host disease. The therapy by combined administration of a GLP-2 derivative and/or a long-acting conjugate thereof and a steroid of the present invention exhibits an improved effect compared to the therapy by single administration of these, and therefore can be usefully used for the prevention, improvement, or treatment of steroid-refractory graft-versus-host disease.

### [Brief Description of Drawings]

FIG. 1 is a diagram illustrating the change in survival rate when a long-acting conjugate of a GLP-2 derivative is administered in a GVHD model induced by allogeneic transplantation;
FIG. 2 is a diagram illustrating the change in GVHD score when a long-acting conjugate of a GLP-2 derivative is administered in a GVHD model induced by allogeneic transplantation;
FIG. 3 is a diagram illustrating the changes in the length of the small intestine (top) and colon (bottom) when a long-acting conjugate of a GLP-2 derivative is administered in a GVHD model induced by allogeneic transplantation;
FIG. 4 is a diagram illustrating the change in survival rate when a long-acting conjugate of a GLP-2 derivative is administered in an SR-GVHD model induced by allogeneic transplantation;
FIG. 5 is a diagram illustrating the change in GVHD score when a long-acting conjugate of a GLP-2 derivative is administered in an SR-GVHD model induced by allogeneic transplantation;
FIG. 6 is a diagram illustrating the change in survival rate by the administration of a long-acting conjugate of a GLP-2 derivative and a steroid in an SR-GVHD model induced by allogeneic transplantation;
FIG. 7 is a diagram illustrating the change in GVHD score by the administration of a long-acting conjugate of a GLP-2 derivative and a steroid in an SR-GVHD model induced by allogeneic transplantation;
FIG. 8 is a diagram comparing the survival rates of groups administered with a long-acting conjugate of a GLP-2 derivative and a steroid, and groups administered with ruxolitinib and a steroid in an SR-GVHD model induced by allogeneic transplantation; and
FIG. 9 is a diagram comparing the GVHD scores of groups administered with a long-acting conjugate of a GLP-2 derivative and a steroid, and groups administered with ruxolitinib and a steroid in an SR-GVHD model induced by allogeneic transplantation.

### [Best Mode for Carrying Out the Invention]

An aspect implementing the present invention is a composition for the prevention, treatment, or improvement of graft-versus-host disease, containing a GLP-2 derivative and/or a long-acting conjugate thereof.

As a specific example, the present invention is a pharmaceutical composition for the prevention or treatment of graft-versus-host disease, containing a GLP-2 derivative and/or a long-acting conjugate thereof.

As another specific example, the present invention is a food composition for the prevention or improvement of graft-versus-host disease, containing a GLP-2 derivative and/or a long-acting conjugate thereof.

Another aspect implementing the present invention is a composition for the prevention, improvement, or treatment of steroid-refractory graft-versus-host disease, containing a GLP-2 derivative and/or a long-acting conjugate thereof.

As a specific example, the present invention is a pharmaceutical composition for the prevention or treatment of steroid-refractory graft-versus-host disease, containing a GLP-2 derivative and/or a long-acting conjugate thereof.

As another specific example, the present invention is a food composition for the prevention or improvement of steroid-refractory graft-versus-host disease, containing a GLP-2 derivative and/or a long-acting conjugate thereof.

Still another aspect implementing the present invention is a therapy for the prevention, improvement, or treatment of steroid-refractory graft-versus-host disease, including using a GLP-2 derivative and/or a long-acting conjugate thereof in combination with a steroid.

Still another aspect implementing the present invention is a composition for the prevention, treatment, or improvement of steroid-refractory graft-versus-host disease, administering a GLP-2 derivative and/or a long-acting conjugate thereof in combination with a steroid.

As a specific example, the present invention is a pharmaceutical composition for the prevention or treatment of steroid-refractory graft-versus-host disease, administering a GLP-2 derivative and/or a long-acting conjugate thereof in combination with a steroid.

As another specific example, the present invention is a food composition for the prevention or improvement of steroid-refractory graft-versus-host disease, administering a GLP-2 derivative and/or a long-acting conjugate thereof in combination with a steroid.

The composition according to any one of the preceding specific examples, in which the present invention contains a GLP-2 derivative in a pharmaceutically effective amount, and the GLP-2 derivative comprises an amino acid sequence represented by the following General Formula 1:
[General Formula 1]

X₁X₂DGSFSDEMNTILDNLAARDFINWLIQTX₃₀ITDX₃₄ (SEQ ID NO: 10)

where,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or Aib (2-aminoisobutyric acid);
X₃₀ is lysine or arginine; and
X₃₄ is one or more arbitrary amino acids or one or more arbitrary amino acids that have undergone modification,
however, a sequence identical to SEQ ID NO: 1 among amino acid sequences represented by General Formula 1 is excluded.

The composition according to any one of the preceding specific examples, in which the GLP-2 derivative comprises an amino acid sequence represented by the following General Formula 2:
[General Formula 2]

X₁X₂DGSFSDEMNTILDNLAARDFINWLIQTX₃₀ITDX₃₄ (SEQ ID NO: 9)

where,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or Aib (2-aminoisobutyric acid);
X₃₀ is lysine or arginine; and
X₃₄ is absent or lysine, arginine, glutamine, histidine, 6-azidolysine, or cysteine,
however, a sequence identical to SEQ ID NO: 1 among amino acid sequences represented by General Formula 2 is excluded.

The composition according to any one of the preceding specific examples, in which, in the GLP-2 derivative,
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine,
   or
(7) X₁ is histidine, X₂ is Aib, X₃₀ is lysine, and X₃₄ is cysteine.

The composition according to any one of the preceding specific examples, in which the GLP-2 derivative comprises an amino acid sequence selected from the group consisting of SEQ ID NOs: 2 to 8.

The composition according to any one of the preceding specific examples, in which the C-terminus of the GLP-2 derivative is unmodified or amidated.

The composition according to any one of the preceding specific examples, in which graft-versus-host disease is acute graft-versus-host disease or chronic graft-versus-host disease.

The composition according to any one of the preceding specific examples, in which the graft-versus-host disease is caused by allogeneic transplantation, and the allogeneic transplantation comprises at least one of hematopoietic stem cell transplantation (HSCT), bone marrow transplantation (BMT), peripheral blood stem cell transplantation (PBSCT), umbilical cord blood transplantation (UCBT), or blood transfusion.

The composition according to any one of the preceding specific examples, in which the composition is administered to a subject together with, before, or after allogeneic transplantation.

The composition according to any one of the preceding specific examples, in which the composition causes one or more of the following: an increase in survival rate, an increase in small intestine length, and an increase in colon length in a subject administered with the composition.

The composition according to any one of the preceding specific examples, in which the GLP-2 derivative is in the form of a long-acting conjugate to which a biocompatible substance capable of increasing the *in vivo* half-life of the GLP-2 derivative is bound.

The composition according to any one of the preceding specific examples, in which the conjugate is represented by the following Chemical Formula 1:

[Chem. 1] X-Lₐ-F

where,
X is a GLP-2 derivative;
L is a linker containing an ethylene glycol repeating unit;
a is 0 or a natural number, but the respective L's are independent of each other when a is 2 or more;
F is an immunoglobulin Fc region; and
"-" is a covalent bond.

The composition according to any one of the preceding specific examples, in which the GLP-2 derivative comprises an amino acid sequence selected from the group consisting of SEQ ID NOs: 2 to 8.

The composition according to any one of the preceding specific examples, in which the C-terminus of the GLP-2 derivative is unmodified or amidated.

The composition according to any one of the preceding specific examples, in which the GLP-2 derivative has an amino acid sequence including SEQ ID NO: 4, L is polyethylene glycol, and the immunoglobulin Fc region is an aglycosylated IgG4 Fc region.

**The** composition according to any one of the preceding specific examples, in which the immunoglobulin Fc region is an aglycosylated IgG4 Fc region.

**The** composition according to any one of the preceding specific examples, in which the F is a dimer composed of two polypeptide chains, and one end of L is linked to only one of the two polypeptide chains.

**The** composition according to any one of the preceding specific examples, in which the L is polyethylene glycol.

**The** composition according to any one of the preceding specific examples, in which the formula weight of the ethylene glycol repeating unit moiety in the L is in a range of 1 to 100 kDa.

The composition according to any one of the preceding specific examples, in which the composition further contains a pharmaceutically acceptable carrier, excipient, or diluent.

The composition according to any one of the preceding specific examples, in which the composition is administered to a subject together with, before, or after allogeneic transplantation.

The composition according to any one of the preceding specific examples, in which the composition causes one or more of the following: an increase in survival rate, an increase in small intestine length, and an increase in colon length in a subject administered with the composition.

The composition according to any one of the preceding specific examples, in which the steroid-refractory graft-versus-host disease is steroid-refractory acute graft-versus-host disease or steroid-refractory chronic graft-versus-host disease.

The composition according to any one of the preceding specific examples, in which the steroid-refractory graft-versus-host disease is caused by allogeneic transplantation, and the allogeneic transplantation comprises at least one of hematopoietic stem cell transplantation (HSCT), bone marrow transplantation (BMT), peripheral blood stem cell transplantation (PBSCT), umbilical cord blood transplantation (UCBT), or blood transfusion.

The composition according to any one of the preceding specific examples, in which the graft-versus-host disease is steroid-refractory graft-versus-host disease, and the pharmaceutical composition is administered in combination with a steroid.

The composition according to any one of the preceding specific examples, in which the steroid-refractory graft-versus-host disease is steroid-refractory acute graft-versus-host disease or steroid-refractory chronic graft-versus-host disease.

The composition according to any one of the preceding specific examples, in which the combined administration is to administer a GLP-2 derivative or a long-acting conjugate thereof; and a steroid in combination simultaneously, sequentially, or in reverse order.

Still another aspect of the present invention is a method for preventing, improving, or treating steroid-refractory graft-versus-host disease, the method including administering a GLP-2 derivative, a long-acting conjugate thereof, and/or a composition containing the same to a subject in need thereof, in which the GLP-2 derivative, the long-acting conjugate thereof, and the composition are as described in any one of the preceding specific examples.

Still another aspect of the present invention is a use of a GLP-2 derivative, a long-acting conjugate thereof, and/or a composition containing the same for the prevention, improvement, or treatment of steroid-refractory graft-versus-host disease, in which the GLP-2 derivative, the long-acting conjugate thereof, and the composition are as described in any one of the preceding specific examples.

Still another aspect of the present invention is a use of a GLP-2 derivative, a long-acting conjugate thereof, and/or a pharmaceutical composition containing the same in the manufacture of a medicament for the prevention or treatment of steroid-refractory graft-versus-host disease, in which the GLP-2 derivative, the long-acting conjugate thereof, and the composition are as described in any one of the preceding specific examples.

Still another aspect implementing the present invention is a pharmaceutical composition for the prevention, improvement, or treatment of steroid-refractory graft-versus-host disease, containing the combination.

Still another aspect implementing the present invention is a pharmaceutical kit for the prevention, improvement, or treatment of steroid-refractory graft-versus-host disease, containing the combination.

Still another aspect implementing the present invention is a method for preventing, improving, or treating steroid-refractory graft-versus-host disease, the method including administering and/or using the combination, pharmaceutical composition, or pharmaceutical kit to a subject in need thereof.

Still another aspect implementing the present invention is a method for preventing, improving, or treating steroid-refractory graft-versus-host disease, the method including administering in combination and/or using in combination a pharmaceutically effective amount of a GLP-2 derivative, a long-acting conjugate thereof, and/or a composition containing the same, and a pharmaceutically effective amount of a composition containing a steroid, or both of these to a subject in need thereof, in which the GLP-2 derivative, the long-acting conjugate thereof, the composition, and the combination are as described in any one of the preceding specific examples.

Still another aspect implementing the present invention is a use of the combination, pharmaceutical composition, or pharmaceutical kit for the prevention, improvement, or treatment of steroid-refractory graft-versus-host disease; and/or a use of the combination, pharmaceutical composition, or pharmaceutical kit in the manufacture of a medicament for the prevention or treatment of steroid-refractory graft-versus-host disease.

### [Detailed Description of the Invention]

Hereinafter, the present invention will be described in more detail.

Meanwhile, each description and each embodiment disclosed in this application can also be applied to other descriptions and other embodiments, respectively. In other words, all combinations of the various elements disclosed in this application fall within the scope of the present invention. Additionally, the scope of the present invention is not limited by the specific description described below.

Additionally, those skilled in the art will recognize, or be able to ascertain using no more than routine experimentation, many equivalents to the specific aspects of the present invention described in this application. Additionally, such equivalents are intended to be included in the present invention.

Throughout this specification, the conventional one-letter and three-letter codes for naturally occurring amino acids are used, as well as the commonly accepted three-letter codes for other amino acids, such as Aib (2-aminoisobutyric acid), _{AZ}K (6-azidolysine), and the like are used. Additionally, amino acids referred to in this specification by abbreviation are described according to the IUPAC-IUB nomenclature.

| | |
|---|---|
| Alanine Ala, A | Arginine Arg, R |
| Asparagine Asn, N | Aspartic acid Asp, D |
| Cysteine Cys, C | Glutamic acid Glu, E |
| Glutamine Gln,Q | Glycine Gly, G |
| Histidine His, H | Isoleucine Ile, I |
| Leucine Leu, L | Lysine Lys, K |
| Methionine Met, M | Phenylalanine Phe, F |
| Proline Pro, P | Serine Ser, S |
| Threonine Thr, T | Tryptophan Trp, W |
| Tyrosine Tyr, Y | Valine Val, V |

An aspect implementing the present invention is a composition for the prevention, improvement, or treatment of graft-versus-host disease, containing a glucagon-like peptide-2 (GLP-2) derivative. Specifically, the composition may be a pharmaceutical composition for the prevention or treatment of graft-versus-host disease.

As a specific example of the present invention, the composition may include, but is not limited to, a pharmaceutical composition for the prevention or treatment of steroid-refractory graft-versus-host disease, containing a GLP-2 derivative.

**In** the present invention, the term "graft-versus-host disease (GVHD)" refers to a disease in which an immune response occurs when T lymphocytes in the peripheral blood or bone marrow of the donor injected during allogeneic transplantation recognize the body of the host (that is, the patient who has received the allogeneic transplantation) as foreign and attack the body. **In** other words, graft-versus-host disease (GVHD) is a disease that is caused by injected living lymphocytes and causes symptoms such as liver dysfunction, skin lesions, jaundice, diarrhea, fever, and pancytopenia due to an immune response, and in severe cases, the patient may die. Specifically, graft-versus-host disease is caused by allogeneic transplantation, here, allogeneic transplantation may include, but is not limited to, at least one of hematopoietic stem cell transplantation (for example, bone marrow transplantation, peripheral blood stem cell transplantation, and umbilical cord blood transplantation) and blood transfusion.

As a specific example of the present invention, the graft-versus-host disease may be, but is not limited to, acute graft-versus-host disease (aGVHD) or chronic graft-versus-host disease (cGVHD).

Chronic graft-versus-host disease usually occurs more than 100 days after allogeneic transplantation and rarely occurs less than 80 days or more than 1 year after transplantation. Chronic graft-versus-host disease has a long latency period, is slow to manifest its effects on the target organ, and is a major cause of late death after allogeneic hematopoietic stem cell transplantation.

Acute graft-versus-host disease usually occurs within 100 days after allogeneic transplantation and is particularly a major complication of allogeneic hematopoietic stem cell transplantation, which usually occurs within 30 to 40 days after allogeneic hematopoietic stem cell transplantation and affects the skin, liver, and gastrointestinal tract. Such acute graft-versus-host disease occurs in three stages: stage 1 occurs before bone marrow transplantation, and is the stage where the patient's tissue is damaged and, in some cases, the secretion of inflammatory cytokines is stimulated by bacterial infection and the like, and antigen-presenting cells are activated by these inflammatory cytokines; stage 2 is the stage where T cells among the transplanted bone marrow cells are activated, and the patient's antigen-presenting cells that have already been activated differentiate T cells into Th1 (T helper 1) cells, ultimately increasing the secretion of cytokines such as IL-2 and IFN-gamma; and stage 3 is the stage where the patient's organs are destroyed, and cytotoxic T cells and natural killer cells are activated by cytokines secreted from activated Th1 cells, and these cells attack the patient's organs, causing graft-versus-host disease.

As a specific example of the present invention, the graft-versus-host disease may be steroid-refractory graft-versus-host disease (SR-GVHD) that does not sufficiently respond to steroids, and may specifically be steroid-refractory acute graft-versus-host disease (SR-aGVHD) or steroid-refractory chronic graft-versus-host disease (SR-cGVHD), but is not limited thereto.

As a specific example of the present invention, steroid-refractory acute graft-versus-host disease (SR-aGVHD) refers to acute graft-versus-host disease following allogeneic hematopoietic stem cell transplantation that does not respond to standard doses of steroids in first-line treatment. Steroid-refractory acute graft-versus-host disease (SR-aGVHD) occurs in 10% to 30% of patients after allogeneic hematopoietic stem cell transplantation and is a disease with a low survival rate.

As a specific example of the present invention, the chronic graft-versus-host disease may be steroid-refractory chronic graft-versus-host disease (SR-cGVHD).

In the present invention, the term "glucagon-like peptide-2" or "GLP-2" is an agonist of the glucagon-like peptide-2 receptor, which may be in the form of a polypeptide or in the form of a long-acting conjugate to which a biocompatible substance capable of increasing the *in vivo* half-life of the polypeptide is bound, but is not limited thereto. In the present invention, GLP-2 comprises not only natural (for example, human) GLP-2, but also derivatives thereof and conjugates thereof. The GLP-2 is an active ingredient contained in the pharmaceutical composition of the present invention and can be contained in the pharmaceutical composition in a pharmaceutically effective amount.

In the present invention, "GLP-2 receptor agonist" refers to a substance that binds to an *in vivo* or isolated human glucagon-like peptide-2 receptor and causes physiological activity equivalent to or similar to that of natural GLP-2. For example, a GLP-2 agonist may include natural GLP-2 or a GLP-2 derivative.

The amino acid sequence of natural GLP-2 is as follows:
GLP-2(1-33)
HADGSFSDEMNTILDNLAARDFINWLIQTKITD (SEQ ID NO: 1)

In the present invention, the "GLP-2 derivative" comprises a peptide having one or more differences in amino acid sequence compared to natural GLP-2, a peptide in which the natural GLP-2 sequence is modified; and/or a mimic of natural GLP-2 having the function of preventing, treating, and/or improving graft-versus-host disease.

Specifically, the GLP-2 derivative of the present invention may have a modification selected from the group consisting of substitution, addition, deletion, modification, and a combination thereof in at least one amino acid in the natural GLP-2 sequence, but is not limited thereto. The amino acids added at this time can also be unnatural amino acids (for example, D-type amino acids), and substitution of unnatural amino acids in addition to natural amino acids is also possible. The added amino acid sequence may be derived from natural GLP-2, but is not limited thereto. In the present invention, the modification of amino acids may mean, but is not limited to, substitution, addition, deletion, or a combination thereof of at least one amino acid; or independently, chemical substitution (for example, alpha-methylation, alpha-hydroxylation, substitution with an azido group), deletion (for example, deamination) and/or modification (for example, N-methylation) of some groups of an amino acid residue.

In a specific embodiment, the GLP-2 derivative of the present invention may exhibit at least 80%, 85%, 90%, 95%, 96%, 97%, 98%, 99%, or more homology (or identity) with natural GLP-2 in amino acid sequence, and/or may be in a form in which some groups of an amino acid residue of the GLP-2 derivative are chemically substituted (for example, alpha-methylation, alpha-hydroxylation, substitution with an azido group), deleted (for example, deamination) and/or modified for example, N-methylation), but is not limited thereto.

In a specific embodiment, the GLP-2 derivative may have, but is not limited to, the N-terminal amino group substituted, deleted, or modified. The GLP-2 derivative of the present invention may be prepared by a method in which the alpha amino group of the N-terminal histidine is deleted, a method in which the N-terminal amino group is substituted with a hydroxyl group or a carboxyl group for synthesis, a method in which the alpha carbon of the N-terminal histidine and the N-terminal amino group bound to the alpha carbon are deleted and only the imidazo-acetyl functional group is left, a method in which the N-terminal amino group is modified with two methyl groups, and the like.

Specifically, the GLP-2 derivative may be imidazoacetyl-deshistidyl-GLP-2 (CA-GLP-2) in which the alpha carbon of the histidine residue that is the first amino acid of the N-terminus and the N-terminal amino group bound to the alpha carbon are deleted, desaminohistidyl GLP-2 (DA-GLP-2) in which the N-terminal amino group of GLP-2 is deleted, β-hydroxy imidazopropionyldeshistidyl GLP-2 (HY-GLP-2) in which the N-terminal amino group of GLP-2 is substituted with a hydroxyl group, N-dimethylhistidyl GLP-2 (DM-GLP-2) in which the N-terminal amino group of GLP-2 is modified with two methyl groups, or β-carboxyimidazopropionyl-deshistidyl GLP-2 (CX-GLP-2) in which the N-terminal amino group of GLP-2 is substituted with a carboxyl group, but is not limited thereto. As a non-limiting example, the substance structures used in the preparation of GLP-2 derivatives are as follows.

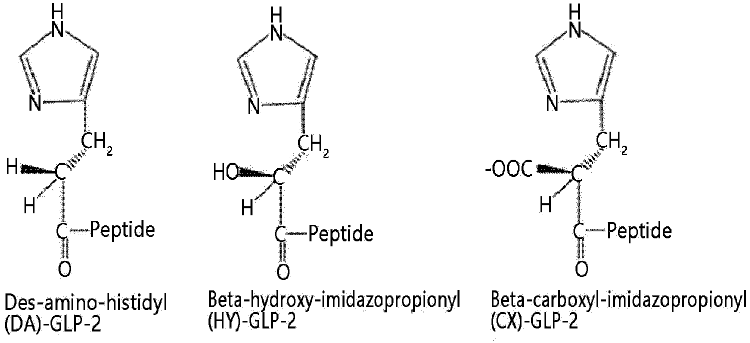

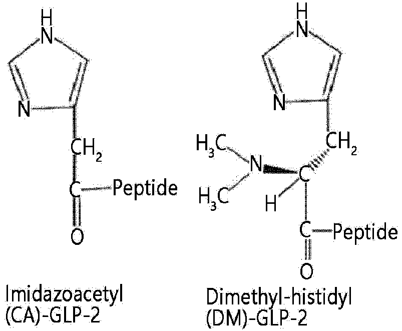

In the present invention, imidazoacetyldeshistidyl(dine), desaminohistidyl(dine), β-hydroxyimidazopropionyldeshistidyl(dine), N-dimethylhistidyl(dine), and β-carboxyimidazopropionyldeshistidyl(dine) may be used with the same meaning as imidazoacetyl, des-amino-histidyl, β-hydroxyimidazopropionyl, dimethyl-histidyl, and β-carboxyl-imidazopropionyl, respectively.

In a specific embodiment, the GLP-2 derivative may include, but is not limited to, a modification in at least one of amino acids 1, 2, 30 and 33 in SEQ ID NO: 1. Specifically, in the present invention, the modification of amino acid may be a modification selected from the group consisting of substitution, addition, deletion, modification and a combination thereof of at least one amino acid, and the amino acid added at this time can be an unnatural amino acid (for example, D-type amino acid), and substitution of an unnatural amino acid in addition to a natural amino acid is also possible. The added amino acid sequence may be derived from natural GLP-2, but is not limited thereto. In the present invention, modification of amino acid may mean, but is not limited to, substitution, addition, deletion, or a combination thereof of at least one amino acid; or independently, chemical substitution (for example, alpha-methylation, alpha-hydroxylation, substitution with an azido group), deletion (for example, deamination) and/or modification (for example, N-methylation) of some groups of an amino acid residue.

In a specific embodiment, the GLP-2 derivative may include, but is not limited to, an amino acid sequence represented by the following General Formula 1:
[General Formula 1]

X₁X₂DGSFSDEMNTILDNLAARDFINWLIQTX₃₀ITDX₃₄ (SEQ ID NO: 10)

where,
X₁ is histidine, imidazoacetyldeshistidine, desaminohistidine, β-hydroxyimidazopropionyldeshistidine, N-dimethylhistidine, or β-carboxyim idazopropionyldeshistidine;
X₂ is alanine, glycine, or Aib (2-aminoisobutyric acid);
X₃₀ is lysine or arginine; and
X₃₄ is one or more arbitrary amino acids or one or more arbitrary amino acids that have undergone modification,
however, a sequence identical to SEQ ID NO: 1 among amino acid sequences represented by General Formula 1 is excluded.

Specifically, the amino acid may be a natural amino acid or an unnatural amino acid, and the modification is as described above.

In a specific embodiment, the GLP-2 derivative may include, but is not limited to, an amino acid sequence represented by the following General Formula 2:
[General Formula 2]

X₁X₂DGSFSDEMNTILDNLAARDFINWLIQTX₃₀ITDX₃₄ (SEQ ID NO: 9)

where,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or Aib (2-aminoisobutyric acid);
X₃₀ is lysine or arginine; and
X₃₄ is absent or lysine, arginine, glutamine, histidine, 6-azidolysine, or cysteine,
however, a sequence identical to SEQ ID NO: 1 among amino acid sequences represented by General Formula 2 is excluded.

Specifically, the GLP-2 derivative of the present invention may have, but is not limited to, a substitution of the second amino acid, alanine, of natural GLP-2 with glycine or Aib (2-aminoisobutyric acid), a substitution of the 30th amino acid, lysine, with arginine, or a combination thereof. The GLP-2 derivative may have a thiol group (for example, cysteine), an amino group (for example, lysine, arginine, glutamine, or histidine), or an azide group (for example, 6-azidolysine) introduced into the C-terminus (for example, 33rd amino acid), but is not limited thereto.

Since binding occurs in the introduced group when a long-acting conjugate of the GLP-2 derivative is prepared, a GLP-2 conjugate with a selectively controlled binding location can be prepared by utilizing this. Specifically, one end of a non-peptide linker may be bound to a hydroxyl group, thiol group, amino group or azide group of the GLP-2 derivative, and a substance (for example, an immunoglobulin Fc region) capable of increasing the *in vivo* half-life may be bound to the other end of the non-peptide linker. The thiol group, amino group or azide group may be introduced by adding an amino acid to GLP-2, but is not limited thereto. The thiol group may be introduced by adding cysteine (C) to GLP-2; the amino group may be introduced by adding lysine (K), arginine (R), glutamine (Q), or histidine (H) to GLP-2; and the azide group may be introduced by adding 6-azidolysine (_{AZ}K) to GLP-2, but is not limited thereto.

Specifically, the GLP-2 derivative may have, but is not limited to, at least one residue being cysteine, lysine, arginine, glutamine, histidine or 6-azidolysine.

Specifically, the GLP-2 derivative of the present invention may include substitution of the second amino acid of natural GLP-2, alanine, with glycine and the introduction of a thiol group (for example, cysteine) into the C-terminus, more specifically may include imidazoacetyldeshistidine in which the alpha carbon of the histidine residue that is the first amino acid at the N-terminus and the N-terminal amino group bound to the alpha carbon are deleted, and may have, for example, an amino acid sequence of SEQ ID NO: 2, but is not limited thereto.

Specifically, the GLP-2 derivative of the present invention may include substitution of the second amino acid of natural GLP-2, alanine, with glycine and the introduction of an amino group (for example, lysine) into the C-terminus, more specifically may include an imidazoacetyldeshistidine in which the alpha carbon of the histidine residue that is the first amino acid of the N-terminus and the N-terminal amino group bound to the alpha carbon are deleted, and may have, for example, an amino acid sequence of SEQ ID NO: 3, but is not limited thereto.

Specifically, the GLP-2 derivative of the present invention may include substitution of the second amino acid of natural GLP-2, alanine, with glycine, substitution of the 30th amino acid of natural GLP-2, lysine, with arginine, and the introduction of an amino group (for example, lysine) into the C-terminus, more specifically may include an imidazoacetyldeshistidine in which the alpha carbon of the histidine residue that is the first amino acid of the N-terminus and the N-terminal amino group bound to the alpha carbon are deleted, and may have, for example, an amino acid sequence of SEQ ID NO: 4, but is not limited thereto.

Specifically, the GLP-2 derivative of the present invention may include substitution of the second amino acid of natural GLP-2, alanine, with glycine and introduction of an azide group (for example, 6-azidolysine) into the C-terminus, more specifically may include an imidazoacetyldeshistidine in which the alpha carbon of the histidine residue that is the first amino acid of the N-terminus and the N-terminal amino group bound to the alpha carbon are deleted, and may have, for example, an amino acid sequence of SEQ ID NO: 5, but is not limited thereto.

Specifically, the GLP-2 derivative of the present invention may include substitution of the second amino acid of natural GLP-2, alanine, with glycine, substitution of the 30th amino acid of natural GLP-2, lysine, with arginine, and the introduction of a thiol group (for example, cysteine) into the C-terminus, more specifically may include an imidazoacetyldeshistidine in which the alpha carbon of the histidine residue that is the first amino acid of the N-terminus and the N-terminal amino group bound to the alpha carbon are deleted, and may have, for example, an amino acid sequence of SEQ ID NO: 6, but is not limited thereto.

Specifically, the GLP-2 derivative of the present invention may include substitution of the second amino acid of natural GLP-2, alanine, with 2-aminoisobutyric acid and introduction of a thiol group (for example, cysteine) into the C-terminus, and may have, for example, an amino acid sequence of SEQ ID NO: 8, more specifically may include an imidazoacetyldeshistidine in which the alpha carbon of the histidine residue that is the first amino acid of the N-terminus and the N-terminal amino group bound to the alpha carbon are deleted, and may have, for example, an amino acid sequence of SEQ ID NO: 7, but is not limited thereto.

The GLP-2 derivatives of SEQ ID NOs: 2 to 8 are presented in Table 1 below.

**[Table 1]**

| Name | Sequence | SEQ ID NO: |
|---|---|---|
| CA GLP-2 KC | *_{ca}*HGDGSFSDEMNTILDNLAARDFINWLIQTKITDC | 2 |
| CA GLP-2 KK | *_{ca}*HGDGSFSDEMNTILDNLAARDFINWLIQTKITDK | 3 |
| CA GLP-2 RK | *_{ca}*HGDGSFSDEMNTILDNLAARDFINWLIQTRITDK | 4 |
| CA GLP-2 K_{AZ}K | *_{ca}*HGDGSFSDEMNTILDNLAARDFINWLIQTKITDAZK | 5 |
| CA GLP-2 RC | *_{ca}*HGDGSFSDEMNTILDNLAARDFINWLIQTRITDC | 6 |
| CA GLP-2 Aib | *_{ca}*HAibDGSFSDEMNTILDNLAARDFINWLIQTKITDC | 7 |
| GLP-2 Aib | HAibDGSFSDEMNTILDNLAARDFINWLIQTKITDC | 8 |

In Table 1, caH indicates histidine substituted with imidazoacetyldeshistidine, Aib indicates 2-aminoisobutyric acid, and _{AZ}K indicates 6-azido-L-lysine.

The GLP-2 derivative according to the present invention may be a peptide including the specific sequence described above, or a peptide (essentially) composed of the specific sequence described above, but is not limited thereto.

Meanwhile, even if it is described in this application as a peptide or GLP-2 derivative 'composed of (consisting of) a specific sequence number', meaningless sequence additions before and after the amino acid sequence of the corresponding sequence number, mutations that may occur naturally, or silent mutations thereof are not excluded as long as a peptide or GLP-2 derivative has the same or equivalent activity as the peptide or GLP-2 derivative consisting of the amino acid sequence of the corresponding sequence number, and it is self-evident that a peptide or GLP-2 derivative falls within the scope of the present invention in a case of having such sequence additions or mutations as well.

Specifically, the GLP-2 derivative may be one represented by General Formula 1 or 2, where (1) X₂ is glycine or Aib, (2) X₃₀ is lysine or arginine, or (3) X₂ is glycine or Aib and X₃₀ is lysine or arginine, but is not limited thereto.

Specifically, the GLP-2 derivative may be one represented by General Formula 1 or 2, where
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine,
   or
(7) X₁ is histidine, X₂ is Aib, X₃₀ is lysine, and X₃₄ is cysteine, but is not limited thereto.

In the present invention, modifications for the preparation of agonists, fragments, variants and derivatives of natural GLP-2 include modifications using L-type or D-type amino acids and/or unnatural amino acids; and/or modifications or post-translational modifications (for example, methylation, acylation, ubiquitination, and intramolecular covalent bonding) of the natural sequence.

In the present invention, the GLP-2 derivative may be in a modified form in which the N-terminus and/or the C-terminus is chemically modified or protected with an organic group, or an amino acid is added to the GLP-2 derivative terminal to protect the GLP-2 derivative from in vivo protein cleavage enzymes and increase the stability thereof.

In particular, in the case of chemically synthesized peptides, since the N- and C-terminuses are charged, the N-terminus may be acetylated and/or the C-terminus may be amidated to remove the charge, but is not particularly limited thereto. Specifically, in the present invention, the GLP-2 derivative may have its C-terminus unmodified or amidated, but is not limited thereto.

The GLP-2 derivative of the present invention may be synthesized by a solid phase synthesis method, can be produced by a recombinant method, and may be manufactured commercially upon request.

In the present invention, the GLP-2 derivative may be in the form of a long-acting conjugate to which a biocompatible substance capable of increasing the *in vivo* half-life of the GLP-2 derivative is bound, but is not limited thereto. The long-acting conjugate may exhibit increased duration of efficacy compared to a GLP-2 derivative to which a biocompatible substance (for example, an immunoglobulin Fc region) is not bound. In the present invention, a conjugate containing a GLP-2 derivative with an increased half-life due to the binding of a biocompatible substance to the GLP-2 derivative is referred to as a " a GLP-2 derivative long-acting conjugate" or a "long-acting conjugate of a GLP-2 derivative." In the present invention, the term "long-acting conjugate" is used interchangeably with "conjugate".

Meanwhile, such a conjugate may be non-naturally occurring.

In the GLP-2 derivative long-acting conjugate, the linkage between a GLP-2 derivative and a biocompatible substance (for example, an immunoglobulin Fc region) may be a physical or chemical bond, or a non-covalent or covalent bond, and may specifically be a covalent bond, but is not limited thereto.

In the GLP-2 derivative long-acting conjugate, the method of linking a GLP-2 derivative and a biocompatible substance (for example, an immunoglobulin Fc region) is not particularly limited, but the GLP-2 derivative and the biocompatible substance may be linked to each other via a linker.

Korean Patent Publication No. 10-2019-0037181 on the GLP-2 derivative long-acting conjugate is incorporated herein by reference.

In a specific embodiment, the long-acting conjugate of the GLP-2 derivative of the present invention may have a structure represented by the following Chemical Formula 1, but is not limited to:

[Chem. 1] X - Lₐ - F

where,
X may be the GLP-2 derivative described herein;
L may be a linker (for example, a linker containing an ethylene glycol repeating unit);
a may be 0 or a natural number, but the respective L's may be independent of each other when a is 2 or more;
F may be a biocompatible substance (for example, an immunoglobulin Fc region) that can increase the *in vivo* half-life of X; and
"-" may be a chemical bond (for example, a covalent bond).

More specifically, X and L, and L and F may be linked to each other by a covalent bond, at this time, the conjugate may be a conjugate in which X, L, and F are each linked through a covalent bond in the order of Chemical Formula 1.

The F may be directly linked to X (for example, a is 0 in Chemical Formula 1) or may be linked via a linker (L).

In the conjugate according to the present invention, X, that is, the GLP-2 derivative, means a GLP-2 derivative described and/or defined herein.

In the conjugate according to the present invention, F is a substance capable of increasing the half-life of X, that is, the GLP-2 derivative according to the present invention, and corresponds to one component of a moiety constituting the conjugate of the present invention.

The F and X may be bound to each other by a covalent chemical bond or a non-covalent chemical bond, and F and X may be bound to each other through L by a covalent chemical bond, a non-covalent chemical bond, or a combination thereof.

The F is a biocompatible substance that can increase the half-life of X, and may be selected from the group consisting of, for example, a high molecular weight polymer, a fatty acid, cholesterol, albumin and fragments thereof, an albumin binding substance, a polymer of a repeating unit of a specific amino acid sequence, an antibody, an antibody fragment, an FcRn binding substance, an *in vivo* connective tissue, a nucleotide, fibronectin, transferrin, a saccharide, heparin, and elastin, but is not particularly limited thereto.

The elastin may be human tropoelastin, which is a water-soluble precursor, may be a polymer of some sequences or some repeating units among these, and, for example, comprises all elastin-like polypeptides, but is not particularly limited thereto.

Examples of the high molecular weight polymer include a high molecular weight polymer selected from the group consisting of polyethylene glycol (PEG), polypropylene glycol, ethylene glycol-propylene glycol copolymer, polyoxyethylated polyol, polyvinyl alcohol, polysaccharides (for example, dextran), polyvinyl ethyl ether, biodegradable polymers, lipid polymers, chitin, hyaluronic acid, oligonucleotides, and any combination thereof, and the polysaccharide may include dextran, but is not particularly limited thereto.

The polyethylene glycol is a term that encompasses, but is not particularly limited to, all forms of ethylene glycol homopolymers, PEG copolymers, or monomethyl-substituted PEG polymers (mPEG).

The biocompatible substance comprises, but is not limited to, poly-amino acids such as poly-lysine, poly-aspartic acid, and poly-glutamic acid.

The fatty acid may be, but is not particularly limited to, those that bind to albumin *in vivo.*

As an example of the F, the F may be an FcRn binding substance, and specifically, the FcRn binding substance may be an immunoglobulin Fc region, more specifically may be an IgG Fc region, still more specifically, an aglycosylated IgG4 Fc region, but is not particularly limited thereto.

As a specific example of the present invention, the F (for example, immunoglobulin Fc region) is a dimer composed of two polypeptide chains, and may have a structure in which one end of L is linked to only one of the two polypeptide chains, but is not limited thereto.

In a specific embodiment, the long-acting conjugate of the present invention may be a GLP-2 derivative linked to an immunoglobulin Fc region, but is not limited thereto.

In the present invention, "immunoglobulin Fc region" means a heavy chain constant region excluding the heavy and light chain variable regions of an immunoglobulin. Specifically, the immunoglobulin Fc region may include a heavy chain constant region 2 (CH2) and/or a heavy chain constant region 3 (CH3) portion, and more specifically may further include a hinge region (the entire hinge region or a portion of the hinge region). The immunoglobulin Fc region may be a component forming a moiety of the conjugate of the present invention. The immunoglobulin Fc region specifically corresponds to F in Chemical Formula 1.

In this specification, the Fc region comprises not only the natural sequence obtained from the papain digestion of immunoglobulin, but also a derivative, a substitution product, and a variant, for example, a sequence in which one or more amino acid residues in the natural sequence are changed by deletion, insertion, non-conservative or conservative substitution, or a combination thereof, and the sequence is thus different from the natural form. It is assumed that the derivative, substitution product, and variant have the ability to bind to FcRn.

The F (for example, an immunoglobulin Fc region) is a structure in which two polypeptide chains are linked by a disulfide bond, and may be a structure in which two polypeptide chains are linked by only the nitrogen atom of one of the two chains, but is not limited thereto. The linkage through a nitrogen atom may be achieved through reductive amination to the epsilon amino atom of lysine or to the N-terminal amino group.

Reductive amination reaction refers to a reaction in which the amine group or amino group of a reactant reacts with the aldehyde of another reactant (that is, a functional group capable of reductive amination) to generate an amine, and then an amine bond is formed through a reduction reaction, and is an organic synthesis reaction that is widely known in the art.

As a specific example, the immunoglobulin Fc region may be linked through its N-terminal nitrogen atom.

Such an immunoglobulin Fc region may include, but is not limited to, a hinge region in the heavy chain constant region.

In the present invention, the immunoglobulin Fc region may include a specific hinge sequence at the N-terminus.

The term "hinge sequence" of the present invention refers to a site that is located in the heavy chain where a dimer of the immunoglobulin Fc region is formed through a disulfide bond.

In the present invention, the hinge sequence may be mutated to have only one cysteine residue by deleting a portion of the hinge sequence having the following amino acid sequence, but is not limited thereto:
Glu-Ser-Lys-Tyr-Gly-Pro-Pro-Cys-Pro-Ser-Cys-Pro (SEQ ID NO: 11).

The hinge sequence may be one in which the 8th or 11th cysteine residue of the hinge sequence of SEQ ID NO: 11 is deleted, thereby containing only one cysteine residue. The hinge sequence of the present invention may be composed of 3 to 12 amino acids, including only one cysteine residue, but is not limited thereto. More specifically, the hinge sequence of the present invention may have the following sequence: Glu-Ser-Lys-Tyr-Gly-Pro-Pro-Pro-Ser-Cys-Pro (SEQ ID NO: 12), Glu-Ser-Lys-Tyr-Gly-Pro-Pro-Cys-Pro-Ser-Pro(SEQ ID NO: 13), Glu-Ser-Lys-Tyr-Gly-Pro-Pro-Cys-Pro-Ser(SEQ ID NO: 14), Glu-Ser-Lys-Tyr-Gly-Pro-Pro-Cys-Pro-Pro(SEQ ID NO: 15), Lys-Tyr-Gly-Pro-Pro-Cys-Pro-Ser(SEQ ID NO: 16), Glu-Ser-Lys-Tyr-Gly-Pro-Pro-Cys(SEQ ID NO: 17), Glu-Lys-Tyr-Gly-Pro-Pro-Cys(SEQ ID NO: 18), Glu-Ser-Pro-Ser-Cys-Pro (SEQ ID NO: 19), Glu-Pro-Ser-Cys-Pro (SEQ ID NO: 20), Pro-Ser-Cys-Pro (SEQ ID NO: 21), Glu-Ser-Lys-Tyr-Gly-Pro-Pro-Ser-Cys-Pro(SEQ ID NO: 22), Lys-Tyr-Gly-Pro-Pro-Ser-Cys-Pro(SEQ ID NO: 23), Glu-Ser-Lys-Tyr-Gly-Pro-Ser-Cys-Pro(SEQ ID NO: 24), Glu-Ser-Lys-Tyr-Gly-Pro-Pro-Cys(SEQ ID NO: 25), Lys-Tyr-Gly-Pro-Pro-Cys-Pro(SEQ ID NO: 26), Glu-Ser-Lys-Pro-Ser-Cys-Pro(SEQ ID NO: 27), Glu-Ser-Pro-Ser-Cys-Pro (SEQ ID NO: 28), Glu-Pro-Ser-Cys (SEQ ID NO: 29), and Ser-Cys-Pro (SEQ ID NO: 30).

Still more specifically, the hinge sequence may include, but is not limited to, an amino acid sequence of SEQ ID NO: 21 (Pro-Ser-Cys-Pro) or SEQ ID NO: 30 (Ser-Cys-Pro).

In a more specific form of the long-acting conjugate of the present invention, the N-terminus of the immunoglobulin Fc region in the conjugate is proline, and this conjugate is one in which the Fc region is linked to a linker via the nitrogen atom of the proline.

In a specific embodiment, the immunoglobulin Fc region may be in a dimeric form in which two chains of the immunoglobulin Fc region form a homodimer or a heterodimer due to the presence of a hinge sequence. The conjugate represented by Chemical Formula 1 of the present invention may be in a form in which one end of the linker is linked to one chain of the immunoglobulin Fc region of the dimer, but is not limited thereto.

The term "N-terminus" of the present invention means the amino terminus of a protein or polypeptide, and may include the very end of the amino terminus, or 1, 2, 3, 4, 5, 6, 7, 8, 9, or 10 or more amino acids from the very end. The immunoglobulin Fc region of the present invention may include a hinge sequence at the N-terminus, but is not limited thereto.

The immunoglobulin Fc region of the present invention may be an extended Fc region including part or all of the heavy chain constant region 1 (CH1) and/or the light chain constant region 1 (CL1), excluding only the heavy and light chain variable regions of the immunoglobulin, as long as it has substantially equivalent or improved effects compared to the natural form. The immunoglobulin Fc may be a region in which a relatively long portion of the amino acid sequence corresponding to CH2 and/or CH3 is deleted.

For example, the immunoglobulin Fc region of the present invention may be 1) a CH1 domain, a CH2 domain, a CH3 domain and a CH4 domain, 2) a CH1 domain and a CH2 domain, 3) a CH1 domain and a CH3 domain, 4) a CH2 domain and a CH3 domain, 5) a combination of one or more of a CH1 domain, CH2 domain, CH3 domain or CH4 domain with an immunoglobulin hinge region (or a part of the hinge region), or 6) a dimer of each domain of a heavy chain constant region and a light chain constant region, but is not limited thereto.

As an embodiment of the long-acting conjugate of the present invention, the immunoglobulin Fc region may be in a dimeric form, and one X molecule may be covalently linked to one Fc region of the dimeric form, at this time, the immunoglobulin Fc and X may be linked to each other by one and the same linker. Meanwhile, it is also possible for two X molecules to bind symmetrically to one Fc region in the dimeric form. At this time, the immunoglobulin Fc and X may be linked to each other by a linker. However, the immunoglobulin Fc region is not limited to the examples described above.

The immunoglobulin Fc region of the present invention comprises not only a natural amino acid sequence but also a sequence derivative thereof. An amino acid sequence derivative means a sequence that has a different sequence from the natural amino acid sequence due to deletion, insertion, non-conservative or conservative substitution of one or more amino acid residues in the natural amino acid sequence, or a combination thereof.

For example, for IgG Fc, amino acid residues 214 to 238, 297 to 299, 318 to 322 or 327 to 331, which are known to be important for binding, may be used as suitable sites for modification.

Various types of derivatives are possible, such as those in which the site capable of forming a disulfide bond may be deleted, those in which several amino acids at the N-terminus of the natural Fc may be deleted, or those in which a methionine residue may be added to the N-terminus of the natural Fc. To eliminate the effector function, complement binding sites, for example, the C1q binding site, may be deleted, or the ADCC (antibody dependent cell mediated cytotoxicity) site may be deleted. Techniques for preparing such sequence derivatives of the immunoglobulin Fc region are disclosed in WO 97/34631, WO 96/32478 and the like.

Amino acid exchanges in proteins and peptides that do not alter the overall activity of the molecule are known in the art (H.Neurath, R.L.Hill, The Proteins, Academic Press, New York, 1979). The most commonly occurring exchange is between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly. In some cases, modification may be achieved by phosphorylation, sulfation, acylation, glycosylation, methylation, farnesylation, acetylation, and amidation.

The an Fc derivative described above exhibits biological activity equivalent to the Fc region of the present invention and may have enhanced structural stability of the Fc region with respect to heat, pH and the like.

Such a Fc region may be obtained from a natural form isolated from living animals such as humans, cows, goats, pigs, mice, rabbits, hamsters, rats or guinea pigs, or may be a recombinant form obtained from a transformed animal cell or microorganism, or a derivative thereof. Here, the method of obtaining the Fc region from a natural form may be a method in which the entire immunoglobulin is isolated from a human or animal living body and then treated with a protease. The immunoglobulin is cleaved into Fab and Fc when treated with papain, and is cleaved into pF'c and F(ab)2 when treated with pepsin. Fc or pF'c can be separated by size-exclusion chromatography and the like. In a more specific embodiment, the Fc region is a recombinant immunoglobulin Fc region obtained from a microorganism, which is a human-derived Fc region.

The immunoglobulin Fc region may be in the form of a natural sugar chain, an increased sugar chain compared to the natural sugar chain, a decreased sugar chain compared to the natural sugar chain, or a form in which the sugar chain has been deleted. Conventional methods such as chemical methods, enzymatic methods, and genetic engineering methods using microorganisms may be used to increase or decrease such an immunoglobulin Fc sugar chain. Here, the immunoglobulin Fc region in which the sugar chain has been deleted from Fc has significantly reduced binding force to complement (C1q), reduced or eliminated antibody-dependent cytotoxicity or complement-dependent cytotoxicity, and thus does not induce unnecessary immune responses *in vivo.* With regard to this, the form that is more suitable for its original purpose as a drug carrier is an immunoglobulin Fc region from which the sugar chain has been deleted or an aglycosylated immunoglobulin Fc region.

In the present invention, "deletion of sugar chains or deglycosylation" refers to an Fc region from which sugar has been deleted by an enzyme, and aglycosylation refers to an Fc region that is produced in a prokaryotic organism, in a more specific embodiment, in *E. coli* and is not glycosylated.

Meanwhile, the immunoglobulin Fc region can be of animal origin, such as human, cow, goat, pig, mouse, rabbit, hamster, rat, or guinea pig, and in a more specific embodiment, of human origin.

The immunoglobulin Fc region may be an Fc region derived from IgG, IgA, IgD, IgE, or IgM, or by a combination thereof, or a hybrid thereof. The immunoglobulin Fc region is derived from IgG or IgM, which is most abundant in human blood in a more specific embodiment, and is derived from IgG, which is known to enhance the half-life of ligand-binding protein in a still more specific embodiment. In a yet still more specific embodiment, the immunoglobulin Fc region is an IgG4 Fc region. In the most specific embodiment, the immunoglobulin Fc region is, but is not limited to, an aglycosylated Fc region derived from human IgG4.

As a specific example, the immunoglobulin Fc region may be a fragment of human IgG4 Fc, which may be a homodimer in which two monomers are linked via a disulfide bond (inter-chain form) between cysteines, the 3rd amino acid of each monomer. At this time, the homodimer has/can have two disulfide bonds (intrachain form), that is, a disulfide bond between cysteines at positions 35 and 95 and between cysteines at positions 141 and 199 in each monomer.

The number of amino acids in each monomer may be composed of 221 amino acids, and the number of amino acids forming a homodimer may be composed of a total of 442 amino acids, but is not limited thereto. Specifically, the immunoglobulin Fc region may be a homodimer (here, two monomers having the amino acid sequence (consisting of 221 amino acids) of SEQ ID NO: 31 form a homodimer through a disulfide bond between cysteines, the 3rd amino acid of each monomer, and the monomers of the homodimer independently form an internal disulfide bond between cysteines at positions 35 and 95 and an internal disulfide bond between cysteines at positions 141 and 199) including the amino acid sequence (consisting of 442 amino acids) of SEQ ID NO: 32, but is not limited thereto.

Meanwhile, in the present invention, the term "combination" in relation to the immunoglobulin Fc region means that a polypeptide encoding a single-chain immunoglobulin Fc region of the same origin forms a bond with a single-chain polypeptide of different origin when a dimer or multimer is formed. In other words, it is possible to prepare a dimer or multimer from two or more Fc regions selected from the group consisting of the Fc regions of IgG Fc, IgA Fc, IgM Fc, IgD Fc, and IgE.

In the present invention, the term "hybrid" means that there are sequences corresponding to two or more immunoglobulin Fc regions of different origins within a single-chain immunoglobulin constant region. In the present invention, various types of hybrids are possible. In other words, a hybrid of domains consisting of one to four domains from the group consisting of CH1, CH2, CH3 and CH4 of IgG Fc, IgM Fc, IgA Fc, IgE Fc and IgD Fc is possible, and may include hinges.

Meanwhile, IgG can also be divided into subclasses of IgG1, IgG2, IgG3, and IgG4, and combinations or hybridizations of these are also possible in the present invention. Specifically, this comprises a subclass of IgG2 and IgG4, and is most specifically, the Fc region of IgG4, which has little effector function such as complement dependent cytotoxicity (CDC).

The conjugate may have an increased duration of efficacy compared to natural GLP-2 or compared to X in which F is not modified, and such a conjugate comprises, but is not limited to, not only the forms described above but also forms encapsulated in biodegradable nanoparticles.

In Chemical Formula 1, the linker L may be a peptide linker or a non-peptide linker (for example, a linker containing an ethylene glycol repeating unit).

When the L is a peptide linker, the peptide linker may contain one or more amino acids, for example, from 1 to 1000 amino acids, but is not particularly limited thereto. In the present invention, in order to link F and X, various known peptide linkers may be used in the present invention, and examples thereof include the [GS]x linker, the [GGGS]x linker, and the [GGGGS]x linker, where x may be a natural number of 1 or more. However, the peptide linker is not limited to these examples.

In the present invention, the "non-peptidyl linker" comprises a biocompatible polymer having two or more repeating units bound thereto. The repeating units are linked to each other via an arbitrary covalent bond rather than a peptide bond. In the present invention, the non-peptide linker contains a reactive group at the end, and can form a conjugate through a reaction with another component that constitutes the conjugate. The non-peptide linker may be a component forming a moiety of the conjugate of the present invention and corresponds to L in Chemical Formula 1.

As the non-peptide linker that can be used in the present invention, any non-peptide polymer that is resistant to *in vivo* proteases can be used without limitation.

In the present invention, the non-peptide linker may be used interchangeably with a non-peptide polymer.

Although not particularly limited thereto, the non-peptide linker may be selected from the group consisting of fatty acids, saccharides, high molecular weight polymers, low molecular weight compounds, nucleotides and combinations thereof. The high molecular weight polymers may be selected from the group consisting of biodegradable polymers such as polyethylene glycol, polypropylene glycol, ethylene glycol-propylene glycol copolymer, polyoxyethylated polyol, polyvinyl alcohol, polysaccharides (for example, dextran), polyvinyl ethyl ether, polylactic acid (PLA) and polylactic-glycolic acid (PLGA), lipid polymers, chitin, hyaluronic acid, oligonucleotides, and combinations thereof.

As the non-peptide linker that can be used in the present invention, any polymer that is resistant to *in vivo* proteases can be used without limitation. Specifically, the molecular weight of the non-peptide polymer may be, but is not limited to, in the range of more than 0 and about 100 kDa, in the range of about 1 to about 100 kDa, specifically in the range of about 1 to about 50 kDa, in the range of about 1 to about 20 kDa, in the range of about 1 to about 10 kDa, or in the range of about 3.4 kDa to 10 kDa.

Although not particularly limited thereto, the non-peptide linker may be linkers containing ethylene glycol repeating units, for example, polyethylene glycol, and derivatives of these already known in the art and derivatives that can be easily prepared within the level of technology in the art are also included in the scope of the present invention.

The repeating unit of the non-peptide linker may be an ethylene glycol repeating unit, specifically, the non-peptide linker may contain an ethylene glycol repeating unit as well as a functional group used in the preparation of a conjugate at the end. The long-acting conjugate according to the present invention may be in a form in which X and F are linked via the functional group, but is not limited thereto. In the present invention, the non-peptide linker may contain two or three or more functional groups, and the respective functional groups may be the same as or different from each other, but is not limited thereto.

Specifically, the linker may be, but is not limited to, polyethylene glycol (PEG) represented by the following Chemical Formula 4: 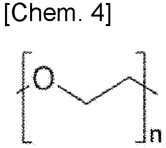 where, n = 10 to 2400, n = 10 to 480, or n = 50 to 250, but is not limited thereto.

In the long-acting conjugate, the PEG moiety may contain, but is not limited to, a -(CH₂CH₂O)ₙ- structure as well as an oxygen atom intervening between the linking element and -(CH₂CH₂O)ₙ-.

In a specific embodiment, the conjugate may be a structure in which a GLP-2 derivative and an immunoglobulin Fc region (F) are covalently linked via a linker containing an ethylene glycol repeating unit, but is not limited thereto.

The polyethylene glycol is a term that encompasses, but is not particularly limited to, all forms of ethylene glycol homopolymers, PEG copolymers, or monomethyl-substituted PEG polymers (mPEG).

As a specific example, the ethylene glycol repeating unit may be represented by, for example, [OCH₂CH₂]ₙ, and the value of n is a natural number and may be determined so that the average molecular weight of the [OCH₂CH₂]ₙ moiety in the peptide conjugate, for example, the number average molecular weight is more than 0 to about 100 kDa, but is not limited thereto. As another example, the value of n is a natural number, and the average molecular weight of the [OCH₂CH₂]ₙ moiety in the peptide conjugate, for example, the number average molecular weight is about 1 to about 100 kDa, about 1 to about 80 kDa, about 1 to about 50 kDa, about 1 to about 30 kDa, about 1 to about 25 kDa, about 1 to about 20 kDa, about 1 to about 15 kDa, about 1 to about 13 kDa, about 1 to about 11 kDa, about 1 to about 10 kDa, about 1 to about 8 kDa, about 1 to about 5 kDa, about 1 to about 3.4 kDa, about 3 to about 30 kDa, about 3 to about 27 kDa, about 3 to about 25 kDa, about 3 to about 22 kDa, about 3 to about 20 kDa, about 3 to about 18 kDa, about 3 to about 16 kDa, about 3 to about 15 kDa, about 3 to about 13 kDa, about 3 to about 11 kDa, about 3 to about 10 kDa, about 3 to about 8 kDa, about 3 to about 5 kDa, about 3 to about 3.4 kDa, about 8 to about 30 kDa, about 8 to about 27 kDa, about 8 to about 25 kDa, about 8 to about 22 kDa, about 8 to about 20 kDa, about 8 to about 18 kDa, about 8 to about 16 kDa, about 8 to about 15 kDa, about 8 to about 13 kDa, about 8 to about 11 kDa, about 8 to about 10 kDa, about 9 to about 15 kDa, about 9 to about 14 kDa, about 9 to about 13 kDa, about 9 to about 12 kDa, about 9 to about 11 kDa, about 9.5 to about 10.5 kDa, about 3.4 kDa or about 10 kDa, but is not limited thereto.

In the present invention, the term "about" is a range that comprises ±0.5, ±0.4, ±0.3, ±0.2, ±0.1, and the like, and comprises all numerical values in a range equal to or similar to the numerical value following the term "about", but is not limited thereto.

As the non-peptide linker of the present invention that is bound to the immunoglobulin Fc region, not only one kind of polymer but also a combination of different kinds of polymers may be used.

In a specific embodiment, both ends of the non-peptide linker may be bound to a thiol group, an amino group, or a hydroxyl group of the immunoglobulin Fc region and a thiol group, an amino group, an azide group, or a hydroxyl group of GLP-2, but are not limited thereto.

Specifically, the non-peptide linker may contain, but is not limited to, a reactive group that can be bound to an immunoglobulin Fc and GLP-2 or a derivative thereof at each of the two ends, specifically, a reactive group that can be bound to a thiol group of cysteine in the immunoglobulin Fc region; an amino group located at the N-terminus, lysine, arginine, glutamine and/or histidine; and/or a hydroxyl group located at the C-terminus, and bound to a thiol group of cysteine of GLP-2; an amino group of lysine, arginine, glutamine and/or histidine; an azide group of azidolysine; and/or a hydroxyl group.

More specifically, the reactive group of the non-peptide polymer may be, but is not limited to, one or more selected from the group consisting of an aldehyde group, a maleimide group, and a succinimide derivative.

In the above, examples of the aldehyde group include, but are not limited to, a propionaldehyde group or a butyraldehyde group.

In the above, as for the succinimide derivative, succinimidyl carboxymethyl, succinimidyl valerate, succinimidyl methylbutanoate, succinimidyl methylpropionate, succinimidyl butanoate, succinimidyl propionate, N-hydroxysuccinimide, hydroxysuccinimidyl or succinimidyl carbonate may be used, but the succinimide derivative is not limited thereto.

The non-peptide linker is linked to a biocompatible substance (for example, immunoglobulin Fc) and a GLP-2 derivative via a reactive group as described above and can be converted into a non-peptide polymer linking portion.

The final product produced by reductive alkylation via an aldehyde bond is much more stable than those linked via an amide bond. The aldehyde reactive group reacts selectively at the N-terminus at a low pH, and can form a covalent bond with a lysine residue at a high pH, for example, pH 9.0.

The terminal reactive groups of the non-peptide linker of the present invention may be the same as or different from each other. The non-peptide linker may have an aldehyde group reactive group at the end, and the non-peptide linker may have an aldehyde group and a maleimide reactive group at the ends, respectively, or may have an aldehyde group and a succinimide reactive group at the ends, respectively, but is not limited thereto.

For example, the non-peptide linker may have a maleimide group at one end and an aldehyde group, a propionaldehyde group or a butyraldehyde group at the other end. As another example, the non-peptide linker may have a succinimidyl group at one end and a propionaldehyde group or a butyraldehyde group at the other end.

In the case of using polyethylene glycol having a hydroxyl reactive group at the propionic end as a non-peptide linker, the conjugate of the present invention can be prepared by activating the hydroxyl group with various reactive groups by known chemical reactions, or by using commercially available polyethylene glycol having modified reactive groups.

In a specific embodiment, the reactive group of the non-peptide linker may be linked to a cysteine residue of GLP-2, more specifically an -SH group of cysteine, but is not limited thereto.

If maleimide-PEG-aldehyde is used, the maleimide group may be linked to the -SH group of the GLP-2 derivative via a thioether bond, and the aldehyde group may be linked to the -NH₂ group of the immunoglobulin Fc through a reductive alkylation reaction, but the non-peptide linker is not limited thereto, and this is just an example.

Through such reductive alkylation, the N-terminal amino group of the immunoglobulin Fc region may be linked to the oxygen atom located at one end of PEG via a linker functional group having the structure of -CH₂CH₂CH₂- to form a structure such as -PEG-O-CH₂CH₂CH₂NH-immunoglobulin Fc, and a structure in which one end of PEG is linked to a sulfur atom located on cysteine of GLP-2 via a thioether bond. The above-described thioether bond may include the structure of

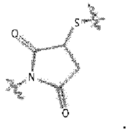

However, the non-peptide linker is not particularly limited to the above-described example, and this is just one example.

In the conjugate, the reactive group of the non-peptide linker may be linked to -NH₂ located at the N-terminus of the immunoglobulin Fc region, but this is an example.

In the conjugate, the GLP-2 derivative may be linked to a non-peptide linker having a reactive group via the C-terminus, but this is an example.

**In** the present invention, the "C-terminus" refers to the carboxyl terminus of a peptide, and for the purpose of the present invention, refers to a location capable of binding to a non-peptide polymer. For example, the C-terminus may include, but is not limited to, not only the most terminal amino acid residue at the C-terminus but also all amino acid residues surrounding the C-terminus, specifically may include the first to twentieth amino acid residues from the very end.

As a specific example, the conjugate represented by Chemical Formula 1 may have a structure represented by the following Chemical Formula 2 or 3.

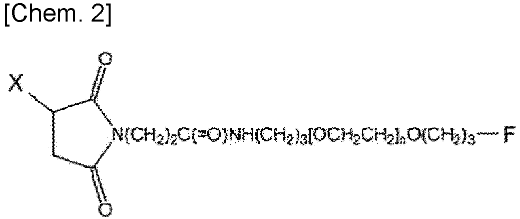

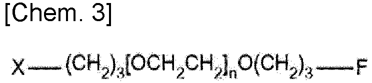

In Chemical Formula 2 or 3, X may be the GLP-2 derivative described above;
F may be an immunoglobulin Fc fragment; and
n may be a natural number. At this time, the description of n is as described above.

As a specific example, the long-acting conjugate represented by Chemical Formula 2 is a structure in which X of the GLP-2 derivative and F of the immunoglobulin Fc region are covalently linked via an ethylene glycol repeating portion, and X may be linked to a succinimide ring of Chemical Formula 2 and F may be linked to an oxypropylene group of Chemical Formula 2, respectively. The long-acting conjugate represented by Chemical Formula 3 is a structure in which X of the GLP-2 derivative and F of the immunoglobulin Fc region are covalently linked via an ethylene glycol repeating portion, and X may be linked to an oxypropylene group of Chemical Formula 3, and F may be linked to another oxypropylene group of Chemical Formula 3, respectively.

As a specific example, in Chemical Formula 2 or 3, the value of n may be determined such that the average molecular weight of the [OCH₂CH₂]ₙ moiety in the conjugate, for example, the number average molecular weight is 1 to 100 kDa, or 1 to 20 kDa, or 10 kDa, but is not limited thereto.

In an embodiment, the succinimide ring of Chemical Formula 2 or the site where X is linked to the succinimide ring of Chemical Formula 2 may be the sulfur atom of the C-terminal cysteine of X. The oxypropylene group of Chemical Formula 3 or the site where X is linked to the oxypropylene group of Chemical Formula 3 may be the sulfur atom of the C-terminal cysteine of X.

The site linked to the oxypropylene group of Chemical Formula 2 or Chemical Formula 3 in F is not particularly limited. In an embodiment of the present invention, the site of F linked to the oxypropylene group may be the N-terminal nitrogen or the nitrogen atom of an internal residue (for example, the epsilon nitrogen of lysine) of F. In a specific embodiment of the present invention, the site where F is linked to the oxypropylene group of Chemical Formula 2 or Chemical Formula 3 may be, but is not limited to, the N-terminal proline of F.

Meanwhile, the GLP-2 derivative or a long-acting conjugate thereof according to the present invention comprises all forms such as itself, a salt of these (for example, a pharmaceutically acceptable salt), and a solvate of these.

The GLP-2 derivative or a long-acting conjugate thereof may be in any pharmaceutically acceptable form.

The kind of salt is not particularly limited. However, it is preferable that the salt be in a form that is safe and effective for a subject, for example, a mammal, but is not particularly limited thereto.

In the present invention, the term "pharmaceutically acceptable salt" comprises a salt derived from a pharmaceutically acceptable inorganic acid, organic acid, or base. Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, and benzenesulfonic acid. Salts derived from suitable bases may include salts of alkali metals such as sodium and potassium, alkaline earth metals such as magnesium, ammonium, and the like.

The term "solvate" used in the present invention refers to a complex formed of a peptide, compound or salt thereof according to the present invention and a solvent molecule.

The GLP-2 derivative of the present invention may be synthesized through a solid phase synthesis method, can be produced by a recombinant method, or can be manufactured commercially upon request, or a commercially available product can be used.

The GLP-2 derivative, a long-acting conjugate thereof, and/or a composition containing the same may be used for the prevention, improvement, or treatment of graft-versus-host disease in a subject. The GLP-2 derivative, a long-acting conjugate thereof, and/or a composition containing the same may be used for the prevention, improvement, or treatment of steroid-refractory graft-versus-host disease in a subject.

The GLP-2 derivative, a long-acting conjugate thereof, and/or a composition containing the same may be used for the prevention, improvement, or treatment of steroid-refractory graft-versus-host disease in a subject, and may be administered in combination with a steroid.

An aspect implementing the present invention is a pharmaceutical composition for the prevention, improvement, or treatment of steroid-refractory graft-versus-host disease, containing a GLP-2 derivative and/or a long-acting conjugate thereof, in which the GLP-2 derivative and/or a long-acting conjugate thereof and a steroid are administered in combination.

Specifically, an aspect of the present invention provides a combination, pharmaceutical composition, or pharmaceutical kit containing a GLP-2 derivative and/or a long-acting conjugate thereof and a steroid. As a specific example, a combination, pharmaceutical composition, or pharmaceutical kit for the prevention, improvement, or treatment of steroid-refractory graft-versus-host disease is provided.

In the present invention, the term "combination" has a use of combined administration of a GLP-2 derivative and/or a long-acting conjugate thereof and a steroid, and may be understood to have the same meaning as "combined use". This also comprises, but is not limited to, the form of a pharmaceutical composition and a pharmaceutical kit in which a GLP-2 derivative and/or a long-acting conjugate thereof and a steroid are used in combination.

Specifically, a GLP-2 derivative and/or a long-acting conjugate thereof and a steroid may be administered in combination simultaneously, sequentially or in reverse order.

The combined administration may be
i) combined administration using a pharmaceutical composition containing a GLP-2 derivative and/or a long-acting conjugate thereof and a steroid in the form of a mixture or in different separated forms, or
ii) to administer a pharmaceutical composition containing a GLP-2 derivative and/or a long-acting conjugate thereof and a pharmaceutical composition containing a steroid in combination simultaneously, sequentially or in reverse order, but is not limited thereto.

The combination or composition may be
a) administered as a mixture containing a GLP-2 derivative and/or a long-acting conjugate thereof and a steroid, or
b) administered in separated forms of a GLP-2 derivative and/or a long-acting conjugate thereof and a steroid, but is not limited thereto.

In the case of separated forms as in b), a GLP-2 derivative and/or a long-acting conjugate thereof and a steroid may be formulated together in different separated forms, respectively; or may be formulated into different preparations, respectively, so that the different preparations are administered simultaneously, individually, sequentially, or in reverse order.

In the present invention, "combined administration," "used in combination," or "using in combination" should be understood not only to mean simultaneous administration, but also to mean an administration form in which a GLP-2 derivative and/or a long-acting conjugate thereof and a steroid act together in a subject so that each substance can perform at a level equivalent to or higher than its original function. Therefore, in a case where the term "combined use" is used herein, it should be understood that the combined use refers to simultaneous, individual, sequential, or reverse administration, and the order is unlimited. In cases where the administration is sequential, reverse or individual, the order of administration is not particularly limited, but the interval between the second and subsequent administrations of ingredients should be such that the beneficial effects of the combined use are not lost.

The preferred dose of the pharmaceutical composition according to the present invention and the ingredient used in combination varies depending on the patient's condition and weight, degree of disease, drug form, route of administration, and period, but may be appropriately selected by those skilled in the art. However, for a desirable effect, the pharmaceutical composition and the ingredient used in combination may be administered at 0.0001 to 500 mg/kg per day, specifically 0.001 to 500 mg/kg. The dose may be administered once a day or divided into several doses and administered. However, the scope of the present invention is not limited by the dose.

In the present invention, the term "composition containing a combination" may be, but is not limited to, the combination containing a GLP-2 derivative and/or a long-acting conjugate thereof and a steroid itself or a composition that contains the combination and has a therapeutic use. For example, the composition may have a use for the prevention, improvement, or treatment of steroid-refractory graft-versus-host disease, but is not limited thereto. In this application, the term "composition containing a combination" may be used interchangeably with "composition".

The composition containing a combination according to the present invention is for administering a GLP-2 derivative and/or a long-acting conjugate thereof in combination with a steroid, and the GLP-2 derivative and/or a long-acting conjugate thereof and the steroid may be formulated into a single preparation.

The term "kit" in the present invention may contain the combination or composition according to the present invention for administering a GLP-2 derivative and/or a long-acting conjugate thereof in combination with a steroid. Specifically, the kit according to the present invention may contain a) a single preparation of a GLP-2 derivative and/or a long-acting conjugate thereof and a steroid; or b) individual preparations of a GLP-2 derivative and/or a long-acting conjugate thereof and a steroid, and may additionally contain substances necessary for the combined administration of two or more substances, but is not limited thereto.

The present invention provides the combined therapy in order to verify that the effect of preventing, improving or treating steroid-refractory graft-versus-host disease is dramatically increased in a case of using a GLP-2 derivative and/or a long-acting conjugate thereof in combination with a steroid compared to a case of using a GLP-2 derivative and/or a long-acting conjugate thereof and a steroid individually.

In the present invention, the term "steroid" is a general term for fat-soluble compounds having a steroid nucleus, such as sterols, bile acids, and sex hormones. A steroid is a lipid and organic compound that does not contain fatty acids and has a common basic structure in which three rings each consisting of six carbon atoms and one ring consisting of five carbon atoms, that is, four rings are bound. Steroids are divided into sugar-regulating steroids and mineral steroids, that is, steroids involved in fluid and blood pressure regulation, depending on their function. Steroids may also be classified into steroids that are synthetic hormones produced naturally in the body and synthetic steroids that are man-made. Examples of steroid hormones include corticosteroids, which include glucocorticoids and mineralocorticoids.

In the present invention, corticosteroids are a drug closely related to cortisol, a hormone naturally produced in the adrenal cortex, and examples thereof include betamethasone, budesonide, cortisone, dexamethasone, hydrocortisone, methylprednisolone, prednisolone, prednisone, deltasone, triamcinolone, mometasone, and fluticasone, but are not limited thereto.

Corticosteroids work by blocking the production of substances that cause allergic and inflammatory reactions, such as prostaglandins, but they interfere with the function of white blood cells, which destroy foreign bodies and enable the immune system to function properly, causing side effects such as increased susceptibility to infection.

Corticosteroids may be used with other medicaments and are prescribed for short-term and long-term use. However, in a case where corticosteroids are used for a long period of time and absorbed into the system, corticosteroids may cause side effects such as appetite and weight gain, fat deposition in the chest, face and the like, water and salt accumulation leading to swelling and edema, high blood pressure, diabetes, osteoporosis, and cataracts.

In the present invention, the steroid comprises a corticosteroid, and the steroid is an active ingredient contained in the pharmaceutical composition of the present invention and may be contained in the pharmaceutical composition in a pharmaceutically effective amount.

The combination, pharmaceutical composition or pharmaceutical kit of the present invention may be used for the prevention, improvement, or treatment of steroid-refractory graft-versus-host disease.

In the present invention, the term "prevention" means any action that completely or partially inhibits or delays the onset of graft-versus-host disease and/or steroid-refractory graft-versus-host disease through the administration of a GLP-2 derivative, a long-acting conjugate of a GLP-2 derivative, and/or a composition containing the same, or a combination or composition containing a GLP-2 derivative and/or a long-acting conjugate thereof and a steroid. The term "treatment" means any action that completely or partially improves or benefits the symptoms of graft-versus-host disease and/or steroid-refractory graft-versus-host disease and comprises the reduction/improvement of symptoms, alleviation of symptom distress, reduction in the incidence of graft-versus-host disease and/or steroid-refractory graft-versus-host disease, or other changes in the patient that increase the response to treatment. The term "improvement" means any action that at least reduces a parameter associated with the condition being treated, for example, the severity of symptoms through the administration of a GLP-2 derivative, a long-acting conjugate thereof, or a composition containing the same.

In the present invention, the term "administration" means introducing a predetermined substance to a patient by any appropriate method, the route of administration of the composition is not particularly limited, but the composition may be administered through any general route through which the composition can reach the target in the living body. For example, the composition may be administered by oral routes or parenteral routes, including intradermal, intravenous, intramuscular, intraarterial, intramedullary, intrathecal, intraventricular, pulmonary, transdermal, subcutaneous, intraperitoneal, intranasal, intragastric, topical, sublingual, vaginal, or rectal routes, but the route of administration is not limited thereto. Meanwhile, since peptides are digested when administered orally, it is desirable that oral compositions are formulated so as to be coated with an active agent or protected from degradation in the stomach. Specifically, the composition of the present invention may be administered in the form of an injection. In the present invention, the composition may be administered by any device capable of transporting the active ingredient to the target cell.

In the present invention, the "subject" refers to a subject requiring allogeneic transplantation, specifically a subject suspected of having graft-versus-host disease and/or steroid-refractory graft-versus-host disease due to allogeneic transplantation, and is a subject that has developed or may develop the disease. As a specific example, the subject may be a mammal, including a human, a mouse, livestock and the like, but comprises any subject treatable with the GLP-2 derivative, a long-acting conjugate thereof, and/or a composition containing the same of the present invention without limitation. A human may be excluded from the subject of the present invention, but the subject is not limited thereto.

In a specific embodiment, the composition of the present invention may be administered to a subject together with, before, or after allogeneic transplantation. For example, the composition of the present invention may be administered within 1 day, 2 days, 3 days, 4 days, 5 days, or 6 days; or within 1 week, 2 weeks, or 1 month before allogeneic transplantation or within 1 day, 2 days, 3 days, 4 days, 5 days, or 6 days; or within 1 week, 2 weeks, or 1 month after allogeneic transplantation, but is not limited thereto.

In a specific embodiment, the composition of the present invention can prevent, treat, or improve graft-versus-host disease and/or steroid-refractory graft-versus-host disease by causing one or more of an increase in survival rate, an increase in small intestine length, and an increase in colon length in a subject administered with the composition, but is not limited thereto.

The pharmaceutical composition of the present invention may additionally contain a pharmaceutically acceptable carrier, excipient or diluent. Such a pharmaceutically acceptable carrier, excipient or diluent may be non-naturally occurring.

In the present invention, the term "pharmaceutically acceptable" means a sufficient amount to exhibit a therapeutic effect and not causing side effects, and can be easily determined by those skilled in the art based on factors well known in the medical field such as the kind of disease, patient's age, weight, health, and sex, patient's sensitivity to drugs, route of administration, method of administration, number of administrations, treatment period, and drugs blended or used simultaneously. The pharmaceutical composition of the present invention may additionally contain a pharmaceutically acceptable excipient. The excipient is not particularly limited thereto, but when administered orally, binders, lubricants, disintegrants, solubilizers, dispersants, stabilizers, suspending agents, pigments, flavoring agents, and the like may be used. In the case of injections, buffers, preservatives, analgesics, solubilizers, isotonic agents, and stabilizers may be used in a mixture. In the case of local administration, bases, excipients, lubricants, and preservatives may be used.

The formulation of the composition of the present invention may be prepared in various ways by mixing the composition with pharmaceutically acceptable excipients as described above. For example, when administered orally, the composition can be prepared in the form of tablets, troches, capsules, elixirs, suspensions, syrups, wafers, and the like. In the case of injections, the composition can be prepared in unit dosage ampoules or multiple dosage forms. The composition can be formulated as solutions, suspensions, tablets, pills, capsules, sustained-release preparations, and the like.

Meanwhile, as examples of carriers, excipients and diluents suitable for formulation, glucose, fructose, galactose, lactose, maltose, dextrose, sucrose, sorbitol, mannitol, xylitol, erythritol, maltitol, starch, amino acids (for example, methionine), acacia, alginate, gelatin, calcium phosphate, calcium silicate, sodium acetate, cellulose, methyl cellulose, microcrystalline cellulose, polysorbate, polyvinylpyrrolidone, water, methyl hydroxybenzoate, propyl hydroxybenzoate, talc, magnesium stearate or mineral oil may be used. The composition may additionally contain fillers, anticoagulants, lubricants, wetting agents, flavoring agents, preservatives, and the like.

The pharmaceutical composition of the present invention may have any one formulation selected from the group consisting of a tablet, a pill, a powder, a granule, a capsule, a suspension, an oral liquid, an emulsion, a syrup, a sterile aqueous solution, a non-aqueous solution, a lyophilized preparation, and a suppository.

The composition may be formulated into a unit dosage form suitable for administration into the patient's body, specifically into a preparation useful for administration of protein drugs according to a conventional method in the pharmaceutical field, and administered by oral routes or parenteral routes, including intradermal, intravenous, intramuscular, intraarterial, intramedullary, intrathecal, intraventricular, pulmonary, transdermal, subcutaneous, intraperitoneal, intranasal, intragastric, topical, sublingual, vaginal or rectal routes, using administration methods commonly used in the art, but is not limited thereto.

A GLP-2 derivative or conjugates thereof can be used by being mixed with various pharmaceutically acceptable carriers, such as saline solution or organic solvents. Carbohydrates such as glucose, sucrose or dextran, antioxidants such as ascorbic acid or glutathione, chelating agents, low molecular weight proteins or other stabilizers, and the like may be used as agents to increase stability or absorbability.

The dose and number of administrations of the pharmaceutical composition of the present invention are determined depending on the kind of drug, which is the active ingredient, along with various related factors, such as the disease to be treated, route of administration, patient's age, sex and weight, and severity of the disease. Specifically, the composition of the present invention may contain the GLP-2 derivative in a pharmaceutically effective amount, but is not limited thereto.

Containing the GLP-2 derivative, a long-acting conjugate thereof, and/or a steroid in a pharmaceutically effective amount means the extent to which the desired pharmacological activity due to the GLP-2 derivative, a long-acting conjugate thereof, and/or a steroid can be attained. This may mean, but is not limited to, a pharmaceutically acceptable level at which no or minimal toxicity or side effects occur in the administered subject. Such a pharmaceutically effective amount may be determined by comprehensively considering the number of administrations, patients, formulation, and the like.

Although not particularly limited thereto, the pharmaceutical composition of the present invention may contain a GLP-2 derivative and/or a long-acting conjugate thereof at 0.01% to 99% weight to volume based on the total volume of the composition, and may contain a pharmaceutically acceptable excipient at 1% to 99.99% weight to volume.

The total effective amount of the composition of the present invention may be administered to a patient in a single dose, and may be administered in multiple doses over a long period of time by a fractionated treatment protocol. The pharmaceutical composition of the present invention may have different contents of the effective ingredient depending on the severity of the disease. Specifically, the preferred total dose of the GLP-2 derivative and/or conjugate of the present invention may be, but is not limited to, about 0.0001 mg to 500 mg per kg of patient body weight per day. However, since the dose of the conjugate is determined as an effective dose for the patient by considering not only the route of administration of the pharmaceutical composition and the number of treatments, but also various factors such as the patient's age, weight, health status, and sex, severity of disease, diet and excretion rate. In consideration of these points, a person of ordinary skill in the art will be able to determine an appropriate effective dose for a specific use of the composition of the present invention. The formulation, route of administration, and method of administration of the pharmaceutical composition according to the present invention are not particularly limited as long as the effects of the present invention are exhibited.

The pharmaceutical composition of the present invention has excellent in *vivo* duration and potency, and thus can significantly reduce the number and frequency of administrations of the pharmaceutical preparation of the present invention, but is not limited thereto.

Another aspect implementing the present invention provides a food composition for the prevention or improvement of graft-versus-host disease, containing a GLP-2 derivative and/or a long-acting conjugate thereof.

Still another aspect implementing the present invention provides a food composition for the prevention or improvement of steroid-refractory graft-versus-host disease, containing a GLP-2 derivative and/or a long-acting conjugate thereof.

The food composition for the prevention or improvement of steroid-refractory graft-versus-host disease is administered in combination with a steroid.

The GLP-2 derivative, long-acting conjugate thereof, steroid, prevention, improvement, graft-versus-host disease or steroid-refractory graft-versus-host disease, and the like are as described above.

The food composition may be used as a health functional food. In cases where the composition of the present invention is used as a food supplementary additive, the peptide (the peptide itself or a long-acting conjugate thereof) may be added as it is or used together with other foods or food ingredients, and the composition may be appropriately used according to a conventional method. The amount of active ingredient mixed may be appropriately determined depending on the intended purpose (prevention, health, or therapeutic treatment).

In the present invention, the term "health functional food" refers to a food manufactured or processed by using a specific ingredient as a raw material or by extracting, concentrating, purifying, mixing, and the like a specific ingredient contained in a food raw material for the purpose of health supplementation, and refers to a food designed and processed so as to sufficiently exert biological regulation functions, such as biological defense, biological rhythm regulation, prevention of the disease and recovery from the disease, on the body by the ingredient. The health food composition can perform functions related to prevention of the disease and recovery from the disease.

Still another aspect implementing the present invention provides a method for preventing, improving, or treating graft-versus-host disease, the method including administering the GLP-2 derivative, a long-acting conjugate thereof, and/or a composition containing the same to a subject in need thereof.

Still another aspect implementing the present invention provides a method for preventing, improving, or treating steroid-refractory graft-versus-host disease, the method including administering the GLP-2 derivative, a long-acting conjugate thereof, and/or a composition containing the same or a composition containing a GLP-2 derivative and/or a long-acting conjugate thereof, and a steroid to a subject in need thereof.

As another specific example, the method may be a method for preventing, improving, or treating graft-versus-host disease and/or steroid-refractory graft-versus-host disease, the method including administering and/or using the combination, pharmaceutical composition or pharmaceutical kit of the present invention to a subject in need thereof.

As still another specific example, the method is a method for preventing, improving, or treating graft-versus-host disease, the method including administering in combination and/or using in combination a pharmaceutically effective amount of a composition containing a GLP-2 derivative and/or a long-acting conjugate thereof to a subject in need thereof, and a method for preventing, improving, or treating steroid-refractory graft-versus-host disease, the method including administering in combination and/or using in combination a pharmaceutically effective amount of a composition containing a steroid to a subject in need thereof.

The GLP-2 derivative, long-acting conjugate thereof, steroid, combined use, composition, subject, prevention, treatment, improvement, graft-versus-host disease and/or steroid-refractory graft-versus-host disease, and the like are as described above.

The method of the present invention may include administering a GLP-2 derivative, a long-acting conjugate thereof, and/or a composition containing the same or a composition containing a GLP-2 derivative and/or a long-acting conjugate thereof and a steroid in a pharmaceutically effective amount. The proper total amount used per day may be determined by the treating physician within the scope of sound medical judgment, and administration may be performed once or several times in divided doses. However, for the purpose of the present invention, it is desirable to apply the specific therapeutically effective amount for a particular patient differently depending on various factors, such as the kind and degree of response to be achieved, the specific composition, including whether other preparations are used in some cases, patient's age, weight, general health status, sex and diet, time of administration, route of administration and secretion rate of the composition, treatment period, and drugs used together with or simultaneously with the specific composition, and similar factors well known in the medical field.

Still another aspect implementing the present invention provides a use of a GLP-2 derivative, a long-acting conjugate thereof, and/or a composition containing the same for the prevention, improvement, or treatment of graft-versus-host disease.

Still another aspect implementing the present invention provides a use of a GLP-2 derivative, a long-acting conjugate thereof, and/or a composition containing the same for the prevention, improvement, or treatment of steroid-refractory graft-versus-host disease.

Still another aspect implementing the present invention provides a use of a composition containing a GLP-2 derivative and/or a long-acting conjugate thereof and a steroid for the prevention, improvement, or treatment of steroid-refractory graft-versus-host disease.

The GLP-2 derivative, long-acting conjugate thereof, composition, steroid, graft-versus-host disease or steroid-refractory graft-versus-host disease, and the like are as described above.

Still another aspect implementing the present invention provides a use of a GLP-2 derivative, a long-acting conjugate thereof, and/or a composition containing the same in the manufacture of a medicament for the prevention, improvement, or treatment of steroid-refractory graft-versus-host disease.

Still another aspect implementing the present invention provides a use of the combination, pharmaceutical composition, or pharmaceutical kit for the prevention, improvement, or treatment of steroid-refractory graft-versus-host disease and/or in the manufacture of a medicament for the prevention, improvement, or treatment of steroid-refractory graft-versus-host disease.

The GLP-2 derivative, long-acting conjugate thereof, prevention, improvement, treatment, steroid, steroid-refractory graft-versus-host disease, combination, pharmaceutical composition, or pharmaceutical kit, and the like are as described above.

Hereinafter, the present invention will be described in more detail by the following Examples. However, the following Examples are only intended to illustrate the present invention and the scope of the present invention is not limited thereto.

### Example 1: Preparation of GLP-2 derivative

In the present invention, seven GLP-2 derivatives having amino acid sequences of SEQ ID NOs: 2 to 8 were prepared, as presented in Table 2 below.

**[Table 2]**

| Name | Sequence | SEQ ID NO: |
|---|---|---|
| CA GLP-2 KC | *_{ca}*HGDGSFSDEMNTILDNLAARDFINWLIQTKITDC | 2 |
| CA GLP-2 KK | *_{ca}*HGDGSFSDEMNTILDNLAARDFINWLIQTKITDK | 3 |
| CA GLP-2 RK | *_{ca}*HGDGSFSDEMNTILDNLAARDFINWLIQTRITDK | 4 |
| CA GLP-2 K_{AZ}K | *_{ca}*HGDGSFSDEMNTILDNLAARDFINWLIQTKITD_{AZ}K | 5 |
| CA GLP-2 RC | *_{ca}*HGDGSFSDEMNTILDNLAARDFINWLIQTRITDC | 6 |
| CA GLP-2 Aib | *_{ca}*HAibDGSFSDEMNTILDNLAARDFINWLIQTKITDC | 7 |
| GLP-2 Aib | HAibDGSFSDEMNTILDNLAARDFINWLIQTKITDC | 8 |

### Example 2: Preparation of GLP-2 derivative long-acting conjugate

As a representative GLP-2 derivative, the GLP-2 derivative of SEQ ID NO: 4 was selected, and in order to pegylate GLP-2 derivative CA GLP-2 RK with modified polyethylene glycol, ALD(2) PEG (modified polyethylene glycol in which hydrogen at both ends is each substituted with a propionaldehyde group (3-oxopropyl group) and the formula weight of the ethylene glycol repeating unit moiety is 3.4 kDa, NOF, Japan), the molar ratio of GLP-2 derivative to ALD(2) PEG was set to 1 : 5 to 1 : 20, the concentration of the GLP-2 derivative was adjusted to 5 to 10 mg/ml, and the reaction was conducted at 2°C to 8°C for 4 to 16 hours. At this time, the reaction was conducted in 20 mM HEPES (pH 7.5) and ethanol, and 20 mM sodium cyanoborohydride was added as a reducing agent for the reaction. The reaction solution was applied to purify a monoPEGylated GLP-2 derivative using the Source 15S (GE, USA) column using a buffer solution containing sodium citrate (pH 2.0) and ethanol and the potassium chloride concentration gradient.

Next, the molar ratio of the purified monoPEGylated GLP-2 derivative to the immunoglobulin Fc fragment was set to 1 : 2 to 1 : 6, the total protein concentration was adjusted to 30 to 35 mg/mL, and the reaction was conducted at 2°C to 8°C for 12 to 20 hours. At this time, the reaction solution was 100 mM potassium phosphate buffer (pH 6.0) and isopropanol, with 20 mM sodium cyanoborohydride added as a reducing agent.

After the reaction was completed, the reaction solution was applied to the Source 15Q (GE, USA) column using a bis-Tris pH 6.5 buffer and the sodium chloride concentration gradient, and applied to the Source 15 ISO (GE, USA) using the concentration gradient of ammonium sulfate and sodium citrate pH 5.0 to 5.2, to purify a long-acting conjugate of the GLP-2 derivative, which is a conjugate in which a GLP-2 derivative is covalently linked to an immunoglobulin Fc fragment via a polyethylene glycol linker.

In addition to the GLP-2 derivative of SEQ ID NO: 4, conjugates of other GLP-2 derivatives of SEQ ID NOs: 2, 3, and 5 to 8 were also prepared by the same method as above.

### Example 3: Examination of effect of treating and preventing acute graft-versus-host disease by administration of GLP-2 derivative long-acting conjugate

In order to construct a mouse allogeneic transplantation model as a graft-versus-host disease (GVHD) model, female donor mice C57BL/6j were purchased from OrientBio (Korea), and female donor and recipient mice BALB/C were purchased from DBL (Korea). The mice were raised in a sterile environment with a 12-h light/dark cycle. Food and water were provided ad libitum, and all procedures were performed under the approval of the Animal Experimentation Committee.

In order to mobilize hematopoietic stem cells (HSCs) into peripheral blood from C57BL/6j or BALB/C mice (donors), 10 µg of G-CSF (granulocyte-colony stimulating factor) (PeproTech, USA) was injected subcutaneously for 5 days, and then 1.5 × 10⁷ splenocytes were collected. The 1.5 × 10⁷ splenocytes (200 µL volume) were injected into BALB/C mice (recipients) through the tail vein.

For transplantation, 7 days before splenocyte injection, busulfan (18.8 mg/kg) (Sigma-Aldrich, USA) was administered to BALB/C mice (recipients) by intraperitoneal injection once a day for 4 days, and subsequently, cyclophosphamide (100 mg/kg) (Sigma-Aldrich, USA) was administered for 2 days.

In order to examine the efficacy of treating and preventing GVHD, the GLP-2 derivative long-acting conjugate prepared in Example 1 was administered to BALB/C mice (recipients) 14 days before splenocyte injection. Specifically, the groups were divided into
- Group 1: BALB/C → BALB/C syngeneic transplantation model - Subcutaneous administration of excipient (n = 10 for survival, n = 4 for histology),
- Group 2: C57BL/6j → BALB/C allogeneic transplantation model - Subcutaneous administration of excipient (n = 10 for survival, n = 5 for histology), and
- Group 3: C57BL/6j → BALB/C allogeneic transplantation model - GLP-2 derivative long-acting conjugate (4,613 µg/kg subcutaneously administered once a week) (n = 11 for survival, n = 5 for histology).

The numerical value expressed as the dose of the GLP-2 derivative long-acting conjugate herein is based on the value acquired by excluding the mass of the polyethylene glycol linker moiety from the total mass of the GLP-2 derivative long-acting conjugate used, that is, the sum of the masses of only the polypeptide moieties.

A composition (for example, a liquid preparation) containing or not containing a GLP-2 derivative long-acting conjugate herein may contain, as excipients, 2 to 200 mM sodium acetate (pH 5.5 to 6.5), 0.002% to 0.2% (w/v) polysorbate, 1% to 20% (w/v) sucrose, and/or 0.01 to 1 mg/mL methionine, and the content of each ingredient may be appropriately adjusted within the above range.

In the GVHD animal model constructed above, the induction of GVHD was evaluated by scoring five medical symptoms: weight loss, activity, posture, fur texture, and skin integrity. Specifically, each of the symptoms was scored from 0 to 2 points, with 10 being the highest score, and the onset signs were evaluated through this (Table 3). In addition, the survival rate was determined, and the small intestine and colon tissues were harvested, and pathological changes were observed.

**[Table 3]**

| Macroscopic evaluation criteria for GVHD onset signs in animal model | | | |
|---|---|---|---|
| **Evaluation items** | **0 point** | **1 point** | **2 points** |
| Weight loss | < 10% | 10% to 25% | > 25% |
| Activity | Normal | Slightly decreased | Move only when stimulated |
| Posture | Normal | Back is bent only when resting | Back is bent so much that movement is impeded |
| Fur texture | Normal | Slightly tangled | Severely tangled |
| Skin integrity | Normal | Toenails are peeling off | Skin is peeling off |

Statistical processing was performed using one-way ANOVA (***p<0.001, **p<0.01, *p<0.05) to compare the control group with the test groups.

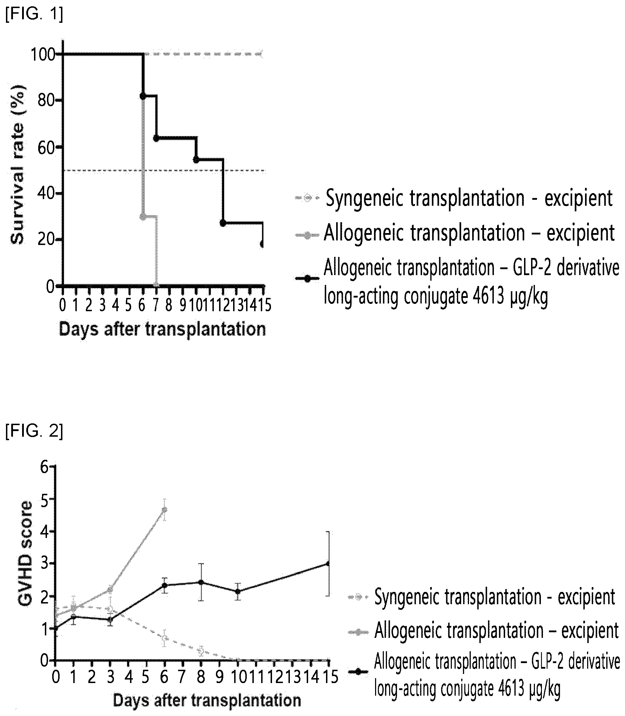

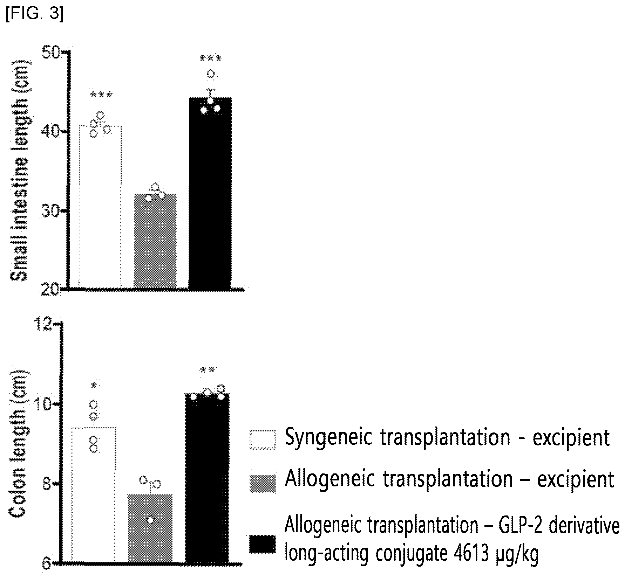

As illustrated in FIG. 1, the allogeneic transplantation mice with induced GVHD (Group 2) exhibited a steadily decreasing survival rate after allogeneic transplantation, with all mice dying within 7 days. However, the GLP-2 derivative long-acting conjugate administration group (Group 3) showed an increased survival rate. It was observed that, in addition to changes in survival rate, mice that underwent allogeneic transplantation displayed clinical signs of acute GVHD, such as weight loss and decreased activity, from day 3. The GLP-2 derivative long-acting conjugate administration group showed improvement or recovery in evaluation items for the onset signs (FIG. 2). As illustrated in FIG. 3, histological findings revealed that allogeneic transplantation mice (Group 2), the control group, exhibited shortened small intestine and colon lengths. However, the GLP-2 derivative long-acting conjugate administration group effectively maintained the small intestine and colon lengths comparable to those of the syngeneic transplantation mice (Group 1).

The results suggest that a GLP-2 derivative long-acting conjugate can exhibit efficacy in preventing and treating graft-versus-host disease.

### Example 4: Examination of effect of treating steroid-refractory graft-versus-host disease acquired by switching from administration of steroid to administration of GLP-2 derivative long-acting conjugate

Steroids are recommended as the first-line treatment for graft-versus-host disease (GVHD), but 40% to 50% of GVHD patients develop steroid-refractory graft-versus-host disease (SR-GVHD), which has a high mortality rate. In the present invention, it was aimed to evaluate the therapeutic effect and responsiveness of a GLP-2 derivative long-acting conjugate for SR-GVHD.

In order to construct a mouse allogeneic transplantation model as a graft-versus-host disease (GVHD) model, female donor mice C57BL/6j were purchased from OrientBio (Korea), and female recipient mice BALB/C were purchased from DBL (Korea). The mice were raised in a sterile environment with a 12-h light/dark cycle. Food and water were provided ad libitum, and all procedures were performed under the approval of the Animal Experimentation Committee.

In order to mobilize hematopoietic stem cells (HSCs) into peripheral blood from 13-week-old C57BL/6j mice (donors), 10 µg of G-CSF (granulocyte-colony stimulating factor) (PeproTech, USA) was injected subcutaneously for 5 days, and then 1.5 × 10⁷ splenocytes were collected. The 1.5 × 10⁷ splenocytes (200 µL volume) were injected into BALB/C mice (recipients) through the tail vein.

For transplantation, 13-week-old BALB/C mice (recipients) were prepared, and 7 days before splenocyte injection, busulfan (18.8 mg/kg) (Sigma-Aldrich, USA) was administered to the mice by intraperitoneal injection once a day for 4 days, and subsequently, cyclophosphamide (100 mg/kg) (Sigma-Aldrich, USA) was administered for 2 days.

In order to examine the responsiveness and therapeutic efficacy for SR-GVHD acquired by switching from administration of a steroid to administration of a GLP-2 derivative long-acting conjugate, splenocytes were injected into the BALB/C mice (recipients), and 3 days later, a GLP-2 derivative long-acting conjugate or a steroid was administered. Specifically
- Group 1: C57BL/6j → BALB/C allogeneic transplantation model - Subcutaneous administration of GLP-2 derivative long-acting conjugate excipient (n = 15)
- Group 2: C57BL/6j → BALB/C allogeneic transplantation model - Apraglutide 469 µg/kg (once every other day, subcutaneously administered) (n = 15)
- Group 3: C57BL/6j → BALB/C allogeneic transplantation model - Prednisolone 2 mg/kg (once a day, intraperitoneally administered for 3 to 16 days) → GLP-2 derivative long-acting conjugate 4,613 µg/kg (once a week, subcutaneously administered instead of steroid from day 17) (n = 15)
- Group 4: C57BL/6j → BALB/C allogeneic transplantation model - GLP-2 derivative long-acting conjugate 4,613 µg/kg (once a week, subcutaneously administered) (n = 15)

The numerical value expressed as the dose of the GLP-2 derivative long-acting conjugate herein is based on the value acquired by excluding the mass of the polyethylene glycol linker moiety from the total mass of the GLP-2 derivative long-acting conjugate used, that is, the sum of the masses of only the polypeptide moieties.

The onset signs were evaluated using the same criteria as in Table 3, the survival rate was determined, and the small intestine and colon tissues were harvested and pathological changes were observed.

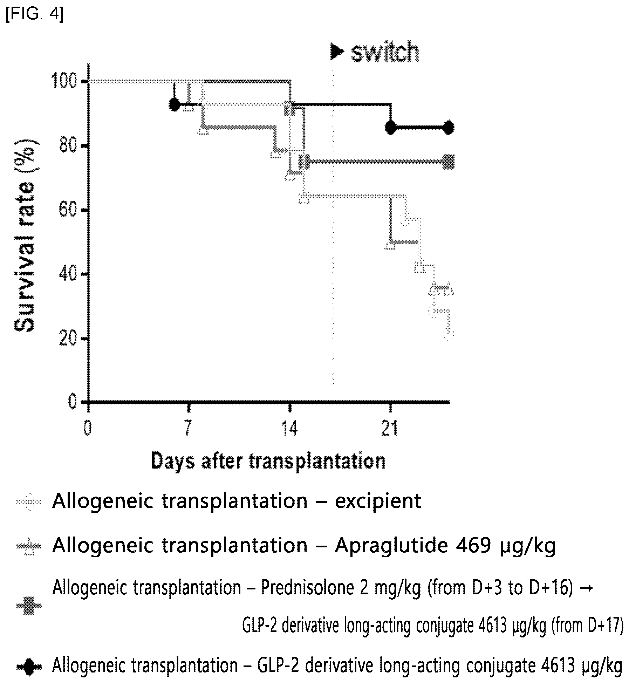

As illustrated in FIG. 4, the allogeneic transplantation mice with induced GVHD (Group 1) had a steadily decreasing survival rate after allogeneic transplantation, and the survival rate on day 25 was 21.4%. The apraglutide administration group (Group 2) had a survival rate of 35.7%, and the GLP-2 derivative long-acting conjugate administration group (Group 4) had an improved survival rate of up to 85.7%. When the survival rate dropped to 75.0% after 16 days of steroid administration, a GLP-2 derivative long-acting conjugate was administered instead of the steroid from day 17 (Group 3), and the survival rate on day 25 was maintained at 75% (FIG. 1).

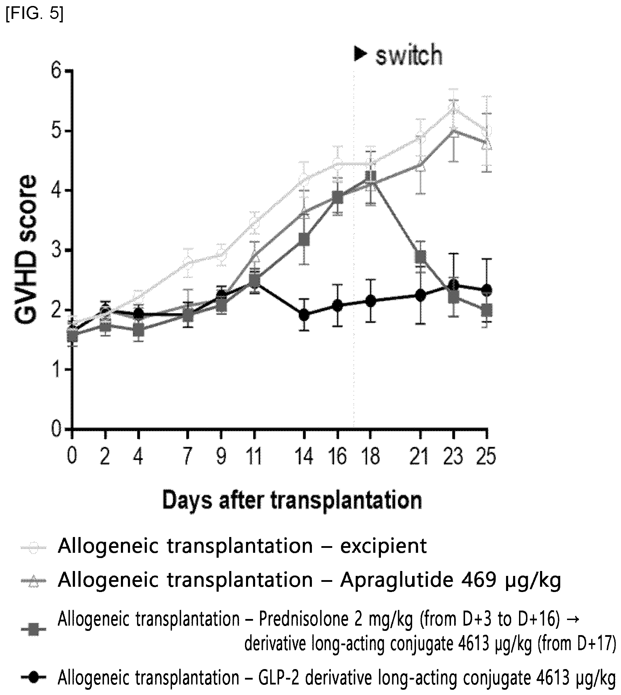

In addition to changes in survival rate, mice that underwent allogeneic transplantation at 13 weeks of age had clinical signs of GVHD, such as weight loss and decreased activity from day 7. The GLP-2 derivative long-acting conjugate administration group maintained clinical scores without worsening of evaluation items in the evaluation of onset signs. The steroid administration group began to show worsening macroscopic conditions by day 16, and was judged to be steroid refractory, and when a GLP-2 derivative long-acting conjugate was administered instead of the steroid from day 17, the evaluation scores showed improvement and recovery (FIG. 5).

The results suggest that a GLP-2 derivative or a long-acting conjugate thereof can exhibit an efficacy of treating steroid-refractory graft-versus-host disease.

### Example 5: Examination of effect of treating steroid-refractory graft-versus-host disease (SR-GVHD) acquired by combined administration of steroid and GLP-2 derivative long-acting conjugate

For graft-versus-host disease (GVHD), steroids have the advantage of being a standard therapeutic agent as both injectable and oral agents, and are used as primary and combined therapeutic agents. Therefore, in the present invention, it was aimed to evaluate the therapeutic effect and responsiveness for SR-GVHD acquired by combined administration of a steroid and a GLP-2 derivative long-acting conjugate.

In order to construct a mouse allogeneic transplantation model as a GVHD model, female donor mice C57BL/6j were purchased from OrientBio (Korea), and female recipient mice BALB/C were purchased from DBL (Korea). The mice were raised in a sterile environment with a 12-h light/dark cycle. Food and water were provided ad libitum, and all procedures were performed under the approval of the Animal Experimentation Committee.

In order to mobilize hematopoietic stem cells (HSCs) into peripheral blood from 12-week-old C57BL/6j mice (donors), 10 µg of G-CSF (granulocyte-colony stimulating factor) (PeproTech, USA) was injected subcutaneously for 5 days, and then 1.5 × 10⁷ splenocytes were collected. The 1.5 × 10⁷ splenocytes (200 µL volume) were injected into BALB/C mice (recipients) through the tail vein.

For a severe GVHD model, 12-week-old BALB/C mice (recipients) were prepared, and 7 days before splenocyte injection, busulfan (18.8 mg/kg) (Sigma-Aldrich, USA) was administered to the mice by intraperitoneal injection once a day for 4 days, and subsequently, cyclophosphamide (100 mg/kg) (Sigma-Aldrich, USA) was administered for 2 days to prepare transplantation.

In order to examine the responsiveness and therapeutic efficacy for SR-GVHD acquired by combined administration of a steroid and a GLP-2 derivative long-acting conjugate, splenocytes were injected into the BALB/C mice (recipients), and 3 days later, a GLP-2 derivative long-acting conjugate or a steroid was administered. Specifically
- Group 1: C57BL/6j → BALB/C allogeneic transplantation model - Subcutaneous administration of GLP-2 derivative long-acting conjugate excipient (n = 15)
- Group 2: C57BL/6j → BALB/C allogeneic transplantation model - Prednisolone 2 mg/kg (once a day, intraperitoneally administered) (n = 15)
- Group 3: C57BL/6j → BALB/C allogeneic transplantation model - GLP-2 derivative long-acting conjugate 4,613 µg/kg (once a week, subcutaneously administered) (n = 15)
- Group 4: C57BL/6j → BALB/C allogeneic transplantation model - Prednisolone 2 mg/kg (once a day, intraperitoneally administered for 3 to 9 days) → GLP-2 derivative long-acting conjugate 4,613 µg/kg (once a week, subcutaneously administered instead of steroid from day 10) (n = 15)
- Group 5: C57BL/6j → BALB/C allogeneic transplantation model - Prednisolone 2 mg/kg (once a day, intraperitoneally administered) + GLP-2 derivative long-acting conjugate 4,613 µg/kg (once a week, subcutaneously administered) (n = 15)

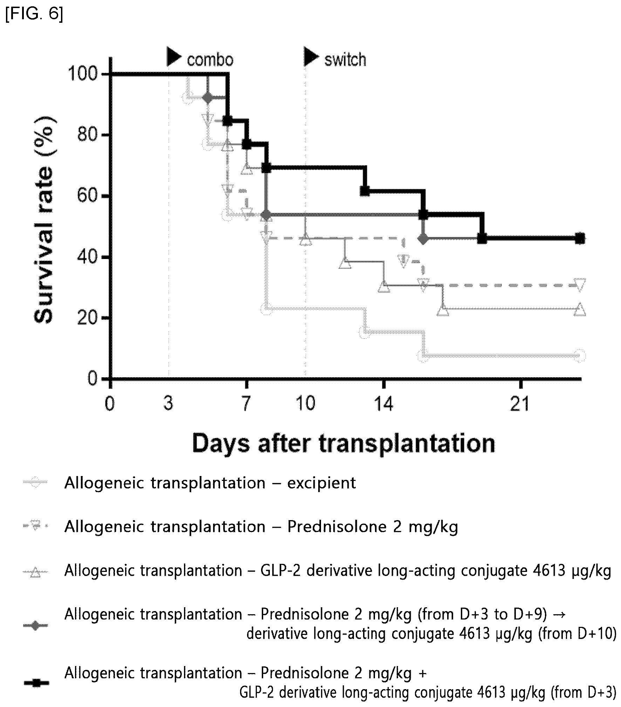

As illustrated in FIG. 6, the allogeneic transplantation mice with induced severe GVHD (Group 1) had a steadily decreasing survival rate after allogeneic transplantation, and the survival rate on day 24 was 7.7%. The survival rate was found to be 30.7% in the steroid administration group (Group 2), and 23.1% in the GLP-2 derivative long-acting conjugate administration group (Group 3). When the survival rate dropped to 53.8% after 9 days of steroid administration, a GLP-2 derivative long-acting conjugate was administered instead of the steroid from day 10 (Group 4), and the survival rate on day 24 was maintained at 46.2%. The survival rate of Group 5, which received combined administration of a steroid and a GLP-2 derivative long-acting conjugate, was 46.2%, and this was superior to that of the steroid only administration group or the GLP-2 derivative long-acting conjugate only administration group.

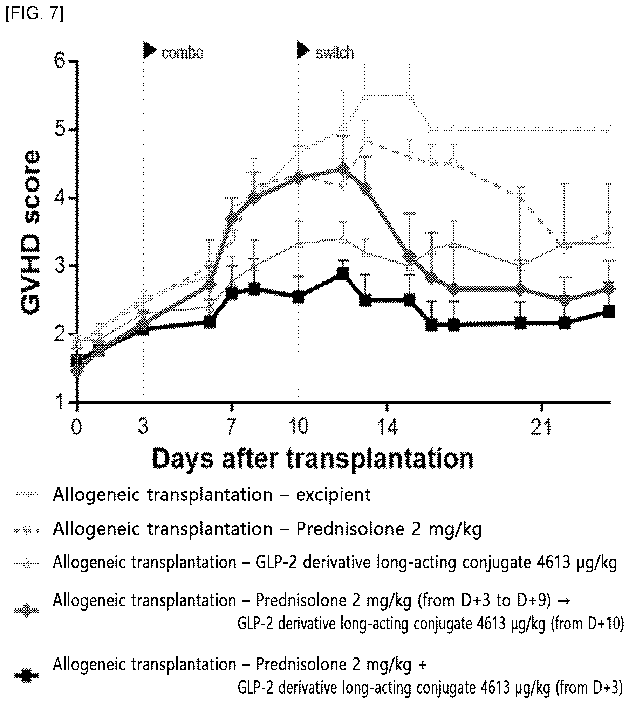

The severity of GVHD was analyzed using clinical scores in mice that underwent allogeneic transplantation at 12 weeks of age to induce severe GVHD. The control allogeneic transplant mice (Group 1) administered with an excipient had clinical signs of GVHD, such as weight loss and decreased activity, after 3 days, and their macroscopic conditions continued to worsen. The GLP-2 derivative long-acting conjugate administration group maintained clinical scores with mild worsening of evaluation items in the onset sign evaluation. The steroid administration group was judged to be steroid refractory as improvement in macroscopic conditions was not observed for 9 days, and when a GLP-2 derivative long-acting conjugate was administered instead of the steroid from day 10, the evaluation scores were improved and the macroscopic conditions showed signs of recovery. In addition, the steroid and GLP-2 derivative long-acting conjugate combined administration group had the most favorable GVHD treating effect, and the evaluation scores were improved compared to those of the steroid only administration group or the GLP-2 derivative long-acting conjugate only administration group (FIG. 7).

### Example 6: Comparison of effect of treating steroid-refractory graft-versus-host disease (SR-GVHD) acquired by combined administration of steroid, GLP-2 derivative long-acting conjugate and/or ruxolitinib

Ruxolitinib is a selective JAK1/2 inhibitor that was first FDA-approved for use in both acute and chronic steroid-refractory graft-versus-host disease (SR-GVHD). Therefore, it was aimed to compare and evaluate the therapeutic effect for SR-GVHD acquired by combined administration of a GLP-2 derivative long-acting conjugate and a steroid with that acquired by combined administration of ruxolitinib and a steroid.

For a severe GVHD model, 12-week-old BALB/C mice (recipients) were prepared, and 7 days before splenocyte injection, busulfan (18.8 mg/kg) (Sigma-Aldrich, USA) was administered to the mice by intraperitoneal injection once a day for 4 days, and subsequently, cyclophosphamide (100 mg/kg) (Sigma-Aldrich, USA) was administered for 2 days to prepare transplantation.

In order to mobilize hematopoietic stem cells (HSCs) into peripheral blood from 12-week-old C57BL/6j mice (donors), 10 µg of G-CSF (granulocyte-colony stimulating factor) (PeproTech, USA) was injected subcutaneously for 5 days, and then 1.5 × 10⁷ splenocytes were collected. The 1.5 × 10⁷ splenocytes (200 µL volume) were injected into BALB/C mice (recipients) through the tail vein.

In order to compare and evaluate the responsiveness and therapeutic efficacy for SR-GVHD acquired by the combined administration of a GLP-2 derivative long-acting conjugate and a steroid with that acquired by the combined administration of ruxolitinib and a steroid, splenocytes were injected into the BALB/C mice (recipients), and 1 day later, a steroid or ruxolitinib or a GLP-2 derivative long-acting conjugate was administered. Specifically
- Group 1: C57BL/6j → BALB/C allogeneic transplantation model - Subcutaneous administration of GLP-2 derivative long-acting conjugate excipient (n = 15)
- Group 2: C57BL/6j → BALB/C allogeneic transplantation model - Prednisolone 2 mg/kg (once a day, intraperitoneally administered) (n = 15)
- Group 3: C57BL/6j → BALB/C allogeneic transplantation model - Ruxolitinib 30 mg/kg (once a day, orally administered) (n = 15)
- Group 4: C57BL/6j → BALB/C allogeneic transplantation model - GLP-2 derivative long-acting conjugate 4,613 µg/kg (once a week, subcutaneously administered) (n = 15)
- Group 5: C57BL/6j → BALB/C allogeneic transplantation model - Prednisolone 2 mg/kg (once a day, intraperitoneally administered) + Ruxolitinib 30 mg/kg (once a day, orally administered) (n = 15)
- Group 6: C57BL/6j → BALB/C allogeneic transplantation model - Prednisolone 2 mg/kg (once a day, intraperitoneally administered) + GLP-2 derivative long-acting conjugate 4,613 µg/kg (once a week, subcutaneously administered) (n = 15)

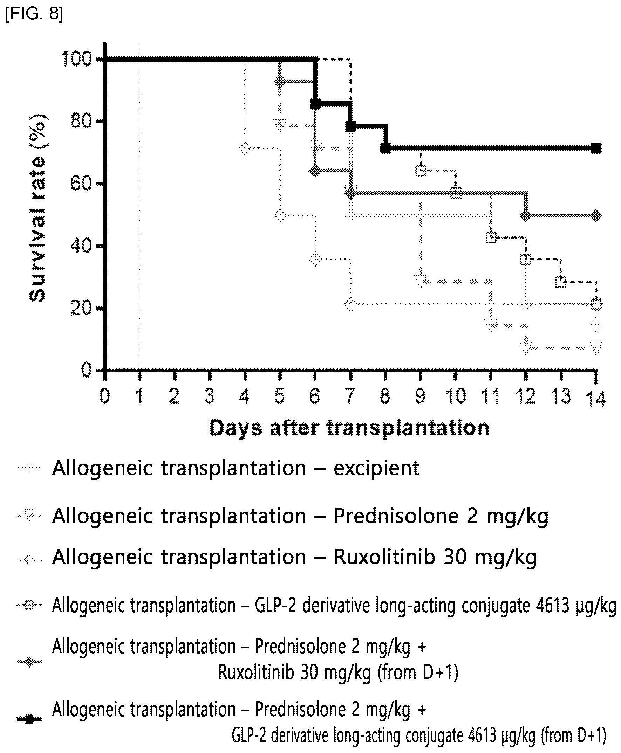

As illustrated in FIG. 8, the allogeneic transplantation mice with induced severe GVHD (Group 1) had a steadily decreasing survival rate after allogeneic transplantation, and the survival rate on day 14 was 14.3%. The survival rate was found to be 7.1% in the steroid only administration group (Group 2), 21.4% in the ruxolitinib only administration group (Group 3), and 21.4% in the GLP-2 derivative long-acting conjugate only administration group (Group 4). The survival rate of the steroid and GLP-2 derivative long-acting conjugate combined administration group (Group 6) was 71.4%, and this was superior to the survival rate of 50.0% of the steroid and ruxolitinib combined administration group (Group 5).

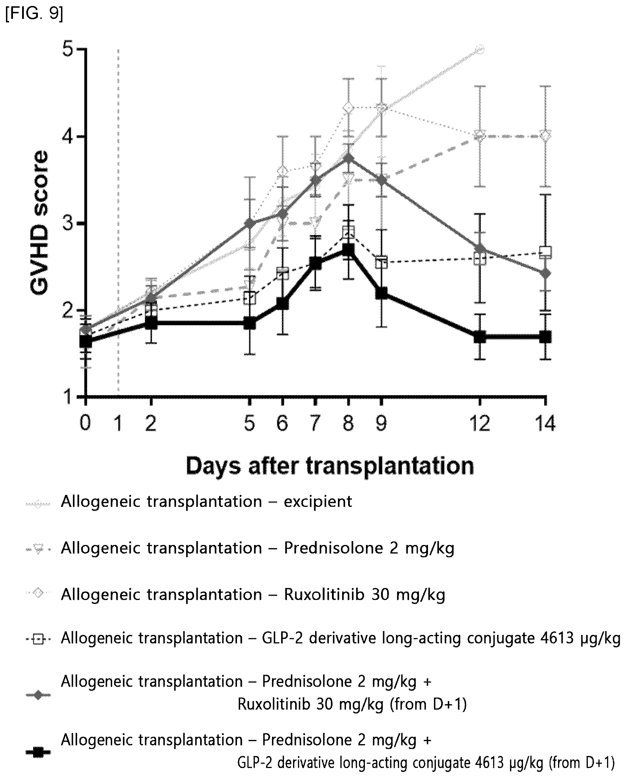

In order to compare the efficacy acquired by combined administration with a steroid in a severe GVHD model, the severity of GVHD was analyzed using clinical scores in mice that underwent allogeneic transplantation. The allogeneic transplant mice (Group 1) administered with an excipient, the control group, had clinical signs of GVHD, such as weight loss and decreased activity, after 2 days, and their macroscopic conditions continued to worsen. In the steroid administration group, the evaluation items did not worsen until day 5, but then rapidly worsened, and the group was judged to be steroid refractory (Group 2). The ruxolitinib only administration group (Group 3) had a continuous deterioration in evaluation items similar to the control group, but the GLP-2 derivative long-acting conjugate only administration group (Group 4) maintained excellent evaluation scores and clinical signs. The steroid and GLP-2 derivative long-acting conjugate combined administration group (Group 6) had superior GVHD evaluation scores compared to the steroid and ruxolitinib combined administration group (Group 5) (FIG. 9).

The results suggest that a GLP-2 derivative or a long-acting conjugate thereof can be used in combination with a steroid, a standard therapeutic agent, to treat steroid-refractory graft-versus-host disease and can exhibit efficacy.

From the above description, those skilled in the art to which the present invention pertains can understand that the present invention can be implemented in other specific forms without changing the technical idea or essential features thereof. In this regard, it should be understood that the embodiments described above are exemplary in all respects and not restrictive. The scope of the present invention is defined by the appended claims rather than by the description preceding them, and therefore all changes or modifications derived from the meaning and scope of the claims and their equivalent concepts should be interpreted as being included in the scope of the present invention.

## Claims

1. A pharmaceutical composition for the prevention or treatment of graft-versus-host disease, comprising a glucagon-like peptide-2 (GLP-2) derivative in a pharmaceutically effective amount, wherein
the GLP-2 derivative comprises an amino acid sequence represented by the following General Formula 1:
[General Formula 1]
X₁X₂DGSFSDEMNTILDNLAARDFINWLIQTX₃₀ITDX₃₄ (SEQ ID NO: 10)
where,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or Aib (2-aminoisobutyric acid);
X₃₀ is lysine or arginine; and
X₃₄ is one or more arbitrary amino acids or one or more arbitrary amino acids that have undergone modification,
however, a sequence identical to SEQ ID NO: 1 among amino acid sequences represented by General Formula 1 is excluded.

2. The pharmaceutical composition according to claim 1, wherein the GLP-2 derivative comprises an amino acid sequence represented by the following General Formula 2:
[General Formula 2]
X₁X₂DGSFSDEMNTILDNLAARDFINWLIQTX₃₀ITDX₃₄ (SEQ ID NO: 9)
where,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or Aib (2-aminoisobutyric acid);
X₃₀ is lysine or arginine; and
X₃₄ is absent or lysine, arginine, glutamine, histidine, 6-azidolysine, or cysteine,
however, a sequence identical to SEQ ID NO: 1 among amino acid sequences represented by General Formula 2 is excluded.

3. The pharmaceutical composition according to claim 2, wherein in the GLP-2 derivative,
(1) X₁ is imidazoacetyldeshistidine, X₂ is glycine, X₃₀ is lysine, and X₃₄ is cysteine,
(2) X₁ is imidazoacetyldeshistidine, X₂ is glycine, X₃₀ is lysine, and X₃₄ is lysine,
(3) X₁ is imidazoacetyldeshistidine, X₂ is glycine, X₃₀ is arginine, and X₃₄ is lysine,
(4) X₁ is imidazoacetyldeshistidine, X₂ is glycine, X₃₀ is lysine, and X₃₄ is 6-azidolysine,
(5) X₁ is imidazoacetyldeshistidine, X₂ is glycine, X₃₀ is arginine, and X₃₄ is cysteine,
(6) X₁ is imidazoacetyldeshistidine, X₂ is Aib, X₃₀ is lysine, and X₃₄ is cysteine,
or
(7) X₁ is histidine, X₂ is Aib, X₃₀ is lysine, and X₃₄ is cysteine.

4. The pharmaceutical composition according to claim 1, wherein the GLP-2 derivative comprises an amino acid sequence selected from the group consisting of SEQ ID NOs: 2 to 8.

5. The pharmaceutical composition according to claim 1, wherein a C-terminus of the GLP-2 derivative is unmodified or amidated.

6. The pharmaceutical composition according to claim 1, wherein the composition causes one or more of an increase in survival rate, an increase in small intestine length, and an increase in colon length in a subject administered with the composition.

7. The pharmaceutical composition according to claim 1, wherein the GLP-2 derivative is in a form of a long-acting conjugate to which a biocompatible substance capable of increasing *in vivo* half-life of the GLP-2 derivative is bound.

8. The pharmaceutical composition according to claim 7, wherein the conjugate is represented by the following Chemical Formula 1:
[Chem. 1] X - Lₐ - F
where,
X is a GLP-2 derivative;
L is a linker containing an ethylene glycol repeating unit;
a is 0 or a natural number, but the respective L's are independent of each other when a is 2 or more;
F is an immunoglobulin Fc region; and
"-" is a covalent bond.

9. The pharmaceutical composition according to claim 8, wherein the GLP-2 derivative comprises an amino acid sequence selected from the group consisting of SEQ ID NOs: 2 to 8.

10. The pharmaceutical composition according to claim 8, wherein a C-terminus of the GLP-2 derivative is unmodified or amidated.

11. The pharmaceutical composition according to claim 8, wherein the immunoglobulin Fc region is an aglycosylated IgG4 Fc region.

12. The pharmaceutical composition according to claim 8, wherein the F is a dimer composed of two polypeptide chains, and one end of L is linked to only one of the two polypeptide chains.

13. The pharmaceutical composition according to claim 8, wherein L is polyethylene glycol.

14. The pharmaceutical composition according to claim 8, wherein a formula weight of an ethylene glycol repeating unit moiety in L is in a range of 1 to 100 kDa.

15. The pharmaceutical composition according to claim 1, wherein the composition further comprises a pharmaceutically acceptable carrier, excipient, or diluent.

16. The pharmaceutical composition according to any one of claims 1 to 15, wherein the composition is administered to a subject together with, before, or after allogeneic transplantation.

17. The pharmaceutical composition according to any one of claims 1 to 15, wherein the graft-versus-host disease is acute graft-versus-host disease or chronic graft-versus-host disease.

18. The pharmaceutical composition according to any one of claims 1 to 15, wherein the graft-versus-host disease is caused by allogeneic transplantation, and the allogeneic transplantation comprises at least one of hematopoietic stem cell transplantation, bone marrow transplantation, peripheral blood stem cell transplantation, umbilical cord blood transplantation, or blood transfusion.

19. The pharmaceutical composition according to any one of claims 1 to 15, wherein the graft-versus-host disease is steroid-refractory graft-versus-host disease.

20. The pharmaceutical composition according to claim 19, wherein the steroid-refractory graft-versus-host disease is steroid-refractory acute graft-versus-host disease or steroid-refractory chronic graft-versus-host disease.

21. The pharmaceutical composition according to any one of claims 1 to 15, wherein the graft-versus-host disease is steroid-refractory graft-versus-host disease, and the pharmaceutical composition is administered in combination with a steroid.

22. The pharmaceutical composition according to claim 21, wherein the steroid-refractory graft-versus-host disease is steroid-refractory acute graft-versus-host disease or steroid-refractory chronic graft-versus-host disease.

23. The pharmaceutical composition according to claim 21, wherein the combined administration is to administer a GLP-2 derivative or a long-acting conjugate thereof; and a steroid in combination simultaneously, sequentially, or in reverse order.
